# EUROPEAN PATENT SPECIFICATION

(11) **EP 1 037 886 B1**
(45) Date of publication and mention of the grant of the patent: **02.05.2003**
(21) Application number: 98963082.7
(22) Date of filing: 11.12.1998
(51) Int. Cl.: C07D 403/04, A61K 31/53, C07D 401/04, C07D 251/18, C07D 417/04, C07D 413/10, C07D 401/12, C07D 409/10, C07D 407/10, C07D 407/04, C07D 409/04, C07D 409/06, C07D 405/04, C07D 453/02, C07D 401/10

(54) **TRIAZINE ANGIOGENESIS INHIBITORS**
TRIAZIN ANGIOGENESE-INHIBITOREN
INHIBITEURS D'ANGIOGENESE A BASE DE TRIAZINE

(30) Priority: 12.12.1997 US 990119; 10.12.1998 US 209396
(43) Date of publication of application: 27.09.2000
(73) Proprietor: Abbott Laboratories, Abbott Park, Illinois 60064-3500 (US)
(72) Inventor: HENKIN, Jack, Highland Park, IL 60035 (US); DAVIDSON, Donald, J., Gurnee, IL 60031 (US); SHEPPARD, George, S., Wilmette, IL 60091 (US); WOODS, Keith, W., Libertyville, IL 60048 (US); MCCROSKEY, Richard, W., Waukegan, IL 60085 (US)
(74) Representative: Modiano, Guido, Dr.-Ing.
(86) International application number: US9826369
(87) International publication number: WO99031088

(56) References cited:
- EP-A- 0 138 209
- EP-A- 0 563 386
- EP-A- 0 976 739
- CA-A- 1 290 754
- DE-A- 2 358 856
- DE-A- 2 919 496
- DE-A- 4 423 138
- IT-A- 924 501
- HORI, TAKAAKI ET AL: "Triazine derivatives inhibit rat hepatocarcinogenesis but do not enhance gap junctional intercellular communication" JPN. J. CANCER RES. (1997), 88(1), 12-17; ISSN: 0910-5050,1997, XP002098384
- VANDERHOEK, RACHAEL ET AL: "Bis(dimethylamino)-s-triazinyl antiinflammatory agents" J. MED. CHEM. (1973), 16(11), 1305-6 , 1973, XP002098385
- DATABASE WPI Section Ch, Week 9249 Derwent Publications Ltd., London, GB; Class B03, AN 92-403404 XP002098387 -& JP 04 300874 A (TSUMURA & CO) , 23 October 1992
- DATABASE WPI Section Ch, Week 9249 Derwent Publications Ltd., London, GB; Class B03, AN 92-403379 XP002098388 -& JP 04 300832 A (TSUMURA & CO) , 23 October 1992
- CHEMICAL ABSTRACTS, vol. 119, no. 3, 19 July 1993 Columbus, Ohio, US; abstract no. 20235, SATO, YASUFUMI ET AL: "Irsogladine is a potent inhibitor of angiogenesis" XP002098386 & FEBS LETT. (1993), 322(2), 155-8 ;ISSN: 0014-5793,1993,

## Description

### Technical Field

The present invention relates to substituted triazines which are useful for treating pathological states which arise from or are exacerbated by angiogenesis, to pharmaceutical compositions comprising these compounds, and to methods of inhibiting angiogenesis in a mammal.

### Background of The Invention

Angiogenesis, the process by which new blood vessels are formed, is essential for normal body activities including reproduction, development and wound repair. Although the process is not completely understood, it is believed to involve a complex interplay of molecules which regulate the growth of endothelial cells (the primary cells of capillary blood vessels). Under normal conditions, these molecules appear to maintain the microvasculature in a quiescent state (i.e. one of no capillary growth) for prolonged periods which may last for as long as weeks or, in some cases, decades. When necessary (such as during wound repair), these same cells can undergo rapid proliferation and turnover within a 5 day period (Folkman, J. and Shing, Y., *The Journal of Biological Chemistry*, 267(16), 10931-10934, **(1992)** and Folkman, J. and Klagsbrun, M., *Science,* 235, 442-447 **(1987).**

Although angiogenesis is a highly regulated process under normal conditions, many diseases (characterized as angiogenic diseases) are driven by persistent unregulated angiogenesis. Otherwise stated, unregulated angiogenesis may either cause a particular disease directly or exacerbate an existing pathological condition. For example, ocular neovascularization has been implicated as the most common cause of blindness and dominates approximately twenty eye diseases. In certain existing conditions, such as arthritis, newly formed capillary blood vessels invade the joints and destroy cartilage. In diabetes, new capillaries formed in the retina invade the vitreous, bleed, and cause blindness. Growth and metastasis of solid tumors are also dependent on angiogenesis (Folkman, J., *Cancer Research*, 46, 467-473 **(1986),** Folkman, J., *Journal of the National Cancer Institute*, 82, 4-6 **(1989).** It has been shown, for example, that tumors which enlarge to greater than 2 mm must obtain their own blood supply and do so by inducing the growth of new capillary blood vessels. Once these new blood vessels become embedded in the tumor, they provide a means for tumor cells to enter the circulation and metastasize to distant sites such as liver, lung or bone (Weidner, N., et al., *The New England Journal of Medicine,* 324(1), 1-8 (**1991**).

Several angiogenesis inhibitors are currently under development for use in treating angiogenic diseases (Gasparini, G. and Harris, A. L., *J*. *Clin. Oncol*., 13(3): 765-782, **1995**), but there are disadvantages associated with these compounds. Suramin, for example, is a potent angiogenesis inhibitor but causes severe systemic toxicity in humans at doses required for antitumor activity. Compounds such as retinoids, interferons and antiestrogens are relatively safe for human use but have weak antiangiogenic effects. Irsogladine, an anti-tumor drug with low toxicity, has only weak anti-angiogenic effects. Thus there is still a need for compounds useful in treating angiogenic diseases in mammals.

As further background art to the present invention, Hori, T. et al. Jpn. J. Cancer Res. 88(1), 12-17 (1997) and Sato, Y. et al., FEBS Letters, Vol. 322, no. 2, 155-158 (1993) discloses irsogladine as an angiogenesis inhibitor. EP-A- 0 563 386 discloses substituted triazines as anticancer agents.

### Summary of the Invention

In one embodiment of the present invention is disclosed the use of a compound of formula (I), 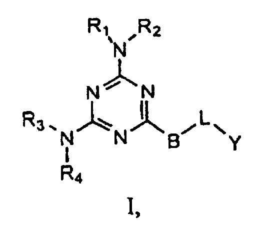 or a pharmaceutically acceptable salt thereof, for manufacturing a medicament for inhibiting angiogenesis in the treatment of cancer, diabetic retinopathy or macular degeneration in a mammal, wherein
R₁, R₂, R₃ and R₄ are independently selected from the group consisting of hydrogen, C₁-C₂₀-alkyl and C₁-C₂₀-alkanoyl;
B is selected from the group consisting of monocyclic aromatic carbocyclic ring, C₃-C₁₀-cycloalkyl, and heterocycle wherein heterocycle is selected from the group consisting of piperidinyl, thiazolyl, furanyl, and thienyl;
Y is selected from the group consisting of monocyclic aromatic carbocyclic ring, C₃-C₁₀-cycloalkyl, and heterocycle wherein heterocycle is selected from the group consisting of piperidinyl, morpholinyl, pyrrolyl, oxazolyl, thienyl, and pyridyl;
wherein the groups defining B and Y can be optionally substituted with 1-3 substituents independently selected from the group consisting of C₁-C₂₀-alkyl, C₁-C₂₀-alkoxy, amino, monocyclic aromatic carbocyclic ring, azido, cyano, halo, C₁-C₂₀-haloalkyl and nitro;
L is selected from the group consisting of a covalent bond, -C(=O)-, C₁-C₂₀-alkylene, C₂-C₂₀-alkenylene, C₂-C₂₀-alkynylene, NHC(O)NH-, -O-, and -S(O)ₜ-, wherein t is 0-2; or wherein said compound is 6-[1-(diphenylmethyl)-3-azetidinyl]-1,3,5-triazine-2,4-diamine.

According to a preferred embodiment, R₁, R₂, R₃ and R₄ are hydrogen. In a further preferred embodiment, L is a covalent bond.

In another embodiment of the invention is disclosed a compound of formula (I), 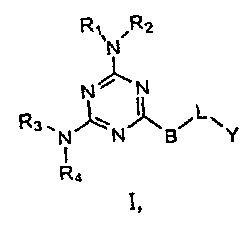 or a pharmaceutically acceptable salt thereof, wherein
R₁, R₂, R₃ and R₄ are independently selected from the group consisting of hydrogen, C₁-C₂₀-alkyl and C₁-C₂₀-alkanoyl;
B is selected from the group consisting of monocyclic aromatic carbocyclic ring, C₃-C₁₀-cycloalkyl, and heterocycle wherein heterocycle is selected from the group consisting of piperidinyl, thiazolyl, furanyl, and thienyl;
Y is selected from the group consisting of monocyclic aromatic carbocyclic ring, C₃-C₁₀-cycloalkyl, and heterocycle wherein heterocycle is selected from the group consisting of piperidinyl, morpholinyl, pyrrolyl, oxazolyl, thienyl, and pyridyl;
wherein the groups defining B and Y can be optionally substituted with 1-3 substituents independently selected from the group consisting of C₁-C₂₀-alkyl, C₁-C₂₀-alkoxy, amino, monocyclic aromatic carbocyclic ring, azido, cyano, halo, C₁-C₂₀-haloalkyl and nitro;
L is selected from the group consisting of a covalent bond, -C(=O)-, C₁-C₂₀-alkylene, C₂-C₂₀-alkenylene, C₂-C₂₀-alkynylene, NHC(O)NH-, -O-, and -S(O)ₜ-,
wherein t is 0-2; or wherein said compound is 6-[1-(diphenylmethyl)-3-azetidinyl]-1,3,5-triazine-2,4-diamine; provided that 2,4-diamino-6-(5-chloro-2-cyclohexyloxy)phenyl)-1,3,5-triazine, 6-(1,1'-biphenyl-3-yl)-1,3,5-triazine-2,4-diamine and 6-(1,1'-biphenyl-4-yl)-1,3,5-triazine-2,4-diamine are excluded.

Also encompassed by the present invention is a compound of formula (I) 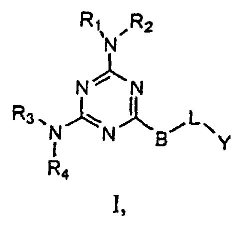 or a pharmaceutically acceptable salt thereof, for use as a therapeutic agent, wherein
R₁, R₂, R₃ and R₄ are independently selected from the group consisting of hydrogen, C₁-C₂₀-alkyl and C₁-C₂₀-alkanoyl;
B is selected from the group consisting of monocyclic aromatic carbocyclic ring, C₃-C₁₀-cycloalkyl, and heterocycle wherein heterocycle is selected from the group consisting of piperidinyl, thiazolyl, furanyl, and thienyl;
Y is selected from the group consisting of monocyclic aromatic carbocyclic ring, C₃-C₁₀-cycloalkyl, and heterocycle wherein heterocycle is selected from the group consisting of piperidinyl, morpholinyl, pyrrolyl, oxazolyl, thienyl, and pyridyl;
wherein the groups defining B and Y can be optionally substituted with 1-3 substituents independently selected from the group consisting of C₁-C₂₀-alkyl, C₁-C₂₀-alkoxy, amino, monocyclic aromatic carbocyclic ring, azido, cyano, halo, C₁-C₂₀-haloalkyl and nitro;
L is selected from the group consisting of a covalent bond, -C(=O)-, C₁-C₂₀-alkylene, C₂-C₂₀-alkenylene, C₂-C₂₀-alkynylene, NHC(O)NH-, -O-, and -S(O)ₜ-,
wherein t is 0-2; or wherein said compound is 6-[1-(diphenylmethyl)-3-azetidinyl]-1,3,5-triazine-2,4-diamine; provided that 2,4-diamino-6-(5-chloro-2-cyclohexyloxy)phenyl)-1,3,5-triazine is excluded.

In one embodiment, R₁, R₂, R₃ and R₄ are hydrogen. In another embodiment, L is a covalent bond.

Compounds of this invention include, but are not limited to, a compound selected from the group consisting of:
6-[1-(diphenylmethyl)-3-azetidinyl]-1,3,5-triazine-2,4-diamine,
6-(1-phenyl-4-piperidinyl)-1,3,5-triazine-2,4-diamine,
trans-6-(4-phenylcyclohexyl)-1,3,5-triazine-2,4-diamine,
6-[3-(1H-pyrrol-1-yl)phenyl]-1,3,5-triazine-2,4-diamine,

6-[1,1'-biphenyl]-2-yl-1,3,5-triazine-2,4-diamine,
6-(4'-nitro[1,1'-biphenyl]-4-yl)-1,3,5-triazine-2,4-diamine,
6-[4-(4-pentylcyclohexyl)phenyl]-1,3,5-triazine-2,4-diamine,
6-(4-phenoxyphenyl)-1,3,5-triazine-2,4-diamine,
*N*-cyclohexyl-N'-[4-(4,6-diamino-1,3,5-triazin-2-yl)phenyl]urea,

*N-*[4-(4,6-diamino-1,3,5-triazin-2-yl)phenyl]-*N*'-phenyl urea,
6-(4'-pentyl[1,1'-biphenyl]-4-yl)-1,3,5-triazine-2,4-diamine,
6-[4'-(pentyloxy)[1,1'-biphenyl]-4-yl]-1,3,5-triazine-2,4-diamine,

6-(4'-amino[1,1'-biphenyl]-4-yl)-1,3,5-triazine-2,4-diamine,
6-[4-(5-oxazolyl)phenyl]-1,3,5-triazine-2,4-diamine,
6-[4-[[5-(trifluoromethyl)-2-pyridinyl]oxy]phenyl]-1,3,5-triazine-2,4-diamine,
4'-(4,6-diamino-1,3,5-triazine-2-yl)[1,1'-biphenyl]-4-carbonitrile,
6-(4'-methoxy[1,1'-biphenyl]-4-yl)-1,3,5-triazine-2,4-diamine,
6-(4'-fluoro[1,1'-biphenyl]-4-yl)-1,3,5-triazine-2,4-diamine

6-[1-([1,1'-biphenyl]-4-yl)-4-piperidinyl]-1,3,5-triazine-2,4-diamine,

6-(1-phenylcyclohexyl)-1,3,5-triazine-2,4-diamine,
6-[1-(4-methoxyphenyl)-4-piperidinyl]-1,3,5-triazine-2,4-diamine,
6-[2-[4-(trifluoromethyl)phenyl]-4-thiazolyl]-1,3,5-triazine-2,4-diamine,
6-[1-(4-methoxyphenyl)cyclohexyl]-1,3,5-triazine-2,4-diamine,
6-[4-(2-thienyl)phenyl]-1,3,5-triazine-2,4-diamine,
6-[4-(phenylethynyl)phenyl]-1,3,5-triazine-2,4-diamine,
*N,N'*-(6-[1,1'-biphenyl]-4-yl-1,3,5-triazin-2,4-diyl)bis[acetamide],
*N*-(4-amino-6-[1,1'-biphenyl]-4-yl-1,3,5-triazin-2-yl)acetamide,

6-(4'-azido[1,1'-biphenyl]-4-yl)-1,3,5-triazine-2,4-diamine,
6-[4-(4-morpholinylsulfonyl)phenyl]-1,3,5-triazine-2,4-diamine,

*N,N'*-[6-(4-phenoxyphenyl)-1,3,5-triazine-2,4-diyl]bis[acetamide],
*N*-[4-amino-6-(4-phenoxyphenyl)-1,3,5-triazin-2-yl]acetamide,
6-(5-phenyl-2-furanyl)-1,3,5-triazine-2,4-diamine,
6-(5-phenyl-2-thienyl)-1,3,5-triazine-2,4-diamine,
*N,N'*-[6-(4-phenylcyclohexyl)-1,3,5-triazin-2,4-diyl]bis[acetamide],
*N*-[4-amino-6-(4-phenylcyclohexyl)-1,3,5-triazin-2-yl]acetamide,

6-[4-(phenylthio)phenyl]-1,3,5-triazine-2,4-diamine,
6-[4-(phenylsulfinyl)phenyl]1,3,5-triazine-2,4-diamine,
6-[4-(phenylsulfonyl)phenyl]-1,3,5-triazine-2,4-diamine,

2,4-diamino-6-[(4-phenylethenyl)phenyl]-1,3,5-triazine,
2,4-diamino-6-[(4-(2-nitrophenyl)ethenyl)phenyl]-1,3,5-triazine,
6-[1,1'-biphenyl]-4-yl-*N*,*N*'-dimethyl-1,3,5-triazine-2,4-diamine,
6-[1,1'-biphenyl]-4-yl-*N*-methyl-1,3,5-triazine-2,4-diamine,

6-[1,1'-biphenyl]-4-yl-*N*,*N*'-diethyl-1,3,5-triazine-2,4-diamine,
6-(2'-nitro[1,1'-biphenyl]-4-yl)-1,3,5-triazine-2,4-diamine,

6-[4-(1-piperidinylsulfonyl)phenyl]-1,3,5-triazine-2,4-diamine,
6-(1-benzoyl-4-piperidinyl)-1,3,5-triazine-2,4-diamine,
6-[1-(phenylmethyl)-4-piperidinyl]-1,3,5-triazine-2,4-diamine,
*N,N'*-diacetyl-6-[4-(phenylsulfonyl)phenyl]-1,3,5-triazine-2,4-diamine,
*N*-acetyl-6-[4-(phenylsulfonyl)phenyl]-1,3,5-triazine-2,4-diamine, and
6-(2-piperidin-1-ylphenyl)-1,3,5-triazine-2,4-diamine,
or a pharmaceutically acceptable salt thereof.

The present invention furthermore includes pharmaceutical compositions comprising a therapeutically effective amount of a compound as defined above and a pharmaceutically acceptable carrier. The present invention also includes the use of a compound as defined above for manufacturing a medicament for inhibiting angiogenesis in the treatment of diabetic retinopathy, macular degeneration or cancer in a mammal.

### Detailed Description of The Invention

### Definition of Terms

The term "alkanoyl" as used herein represents an alkyl group of 1-20 carbon atoms attached to the parent molecular group through a carbonyl group.

The term "alkoxy" as used herein represents an alkyl group of 1-20 carbon atoms attached to the parent molecular group through an oxygen atom.

The term "alkyl" as used herein represents a monovalent group of 1-20 carbon atoms derived from a straight or branched chain saturated hydrocarbon.

The term "alkylene" as used herein represents a saturated divalent group of 1-20 carbon atoms derived from a straight or branched chain saturated hydrocarbon.

The term "alkenylene" as used herein represents an unsaturated divalent group of 2-20 carbon atoms derived from a straight or branched chain alkene.

The term "alkynylene" as used herein represents an unsaturated divalent group of 2-20 carbon atoms derived from a straight or branched chain alkyne.

The term "amino" as used herein represents -NH₂.

The term "azido" as used herein represents -N₃.

The term "cyano" as used herein represents -CN.

The term "cycloalkyl" as used herein represents a saturated monovalent group of 3-10 carbon atoms derived from a cyclic or bicyclic hydrocarbon.

The term "halo" as used herein represents F, Cl, Br, or I.

The term "haloalkyl" as used herein represents an alkyl group to which is attached at least one halogen atom.

The term "nitro" as used herein represents -NO₂.

The term "pharmaceutically acceptable prodrugs" as used herein represents those prodrugs of the compounds of the present invention which are, within the scope of sound medical judgement, suitable for use in contact with with the tissues of humans and lower animals with undue toxicity, irritation, allergic response, and the like, commensurate with a reasonable benefit/risk ratio, and effective for their intended use, as well as the zwitterionic forms, where possible, of the compounds of the invention.

Pharmaceutically acceptable prodrugs of the compounds of the invention can be rapidly transformed in vivo to the parent compound of the above formula, for example, by hydrolysis in blood. A thorough discussion is provided in T. Higuchi and V. Stella, Prodrugs as Novel Delivery Systems, Vol. 14 of the A.C.S. Symposium Series, and in Edward B. Roche, ed., Bioreversible Carriers in Drug Design, American Pharmaceutical Association and Pergamon Press, 1987.

Asymmetric or chiral centers may exist in the compounds of the present invention. The present invention contemplates the various stereoisomers and mixtures thereof. Individual stereoisomers of compounds of the present invention are prepared synthetically from commercially available starting materials which contain asymmetric or chiral centers or by preparation of mixtures of enantiomeric compounds followed by resolution well-known to those of ordinary skill in the art. These methods of resolution are exemplified by (1) attachment of a racemic mixture of enantiomers, designated (+/-), to a chiral auxiliary, separation of the resulting diastereomers by recrystallization or chromatography and liberation of the optically pure product from the auxiliary or (2) direct separation of the mixture of optical enantiomers on chiral chromatographic columns. Pure enantiomers are designated herein by the symbols "R" or "S," depending on the configuration of subsitiuents around the chiral carbon atom.

Geometric isomers may also exist in the compounds of the present invention. The present invention contemplates the various geometric isomers and mixtures thereof resulting from the arrangement of substituents around a carbon-carbon double bond or arrangement of substituents around a carbocyclic ring. Substituents around a carbon-carbon double bond are designated as being in the Z or E configuration wherein the term "Z" represents substituents on the same side of the carbon-carbon double bond and the term "E" represents substituents on opposite sides of the carbon-carbon double bond. The arrangement of substituents around a carbocyclic ring are designated as cis or trans wherein the term "cis" represents substituents on the same side of the plane of the ring and the term "trans" represents substituents on opposite sides of the plane of the ring. Mixtures of compounds wherein the substitutients are disposed on both the same and opposite sides of plane of the ring are designated cis/trans.

### Endothelial Cell Migration Assay

The endothelial cell migration assay was performed essentially as described by Polverini, P.J. et al., *Methods Enzymol*, 198: 440-450 **(1991).** Briefly, Human Microvascular Endothelial Cells (HMVEC) were starved overnight in DMEM (Dulbecco's Modified Eagle Medium) containing 0.1% bovine serum albumin (BSA). Cells were then harvested with trypsin and resuspended in DMEM with 0.1% BSA at a concentration of 1.5 x 10⁶ cells/mL. Cells were added to the bottom of a 48-well modified Boyden chamber (Nucleopore Corporation, Cabin John, MD). The chamber was assembled and inverted, and cells were allowed to attach for 2 hours at 37 °C to polycarbonate chemotaxis membranes (5 µm pore size) that had been soaked in 0.1% gelatin overnight and dried. The chamber was then reinverted and basic fibroblast growth factor (bFGF) and test substances were added to the wells of the upper chamber (to a total volume of 50 µL); the apparatus was then incubated for 4 hours at 37 °C. Membranes were recovered, fixed and stained (DiffQuick, Fisher Scientific, Pittsburgh, PA) and the number of cells that had migrated to the upper chamber per 10 high power fields were counted. Background migration to DMEM + 0.1% BSA was subtracted and the data reported as the number of cells migrated per 10 high power fields (400X) or when results from multiple experiments were combined, as the percent inhibition of migration compared to a positive control. The results are shown in Table 1.

**Table 1**

| Inhibitory Potencies Against bFGF Induced Human Microvascular Endothelial Cell Migration of Representative Compounds | |
|---|---|
| Example | % inhibition at (nM) |
| Irsogladine | 53 % (600 nM) |
| 1 | 20(600) |
| 3 | 100 (600) |
| 4 | 62 (600) |
| 6 | 95 (600) |
| 7 | 100 (600) |
| 8 | 30 (600) |
| 9 | 29 (600) |
| 10 | 29 (600) |
| 12 | 36(600) |
| 13 | 53 % (600 nM) |
| 47 | 65 (600) |
| 48 | 55 (600) |
| 49 | 14 (600) |
| 50 | 100 (600) |
| 51 | 100 (600) |
| 52 | 100 (600) |
| 53 | 85 (600) |
| 55 | 84 (600) |
| 56 | 30 (600) |
| 58 | 100 (600) |
| 60 | 100 (600) |
| 63 | 79 (500) |
| 65 | 32 (200) |
| 68 | 73 (500) |
| 69 | 39 (500) |
| 74 | 82 (500) |
| 75 | 16 (500) |
| 76 | 33 (500) |
| 77 | 50 (500) |

The compounds of the invention, including but not limited to those specified in the examples, possess anti-angiogenic activity. As angiogenesis inhibitors, such compounds are useful in the treatment of both primary and metastatic solid tumors and carcinomas of the breast; colon; rectum; lung; oropharynx; hypopharynx; esophagus; stomach; pancreas; liver; gallbladder; bile ducts; small intestine; urinary tract including kidney, bladder and urothelium; female genital tract including cervix, uterus, ovaries, choriocarcinoma and gestational trophoblastic disease; male genital tract including prostate, seminal vesicles, testes and germ cell tumors; endocrine glands including thyroid, adrenal, and pituitary; skin including hemangiomas, melanomas, sarcomas arising from bone or soft tissues and Kaposi's sarcoma; tumors of the brain, nerves, eyes, and meninges including astrocytomas, gliomas, glioblastomas, retinoblastomas, neuromas, neuroblastomas, Schwannomas and meningiomas; solid tumors arising from hematopoietic malignancies such as leukemias and including chloromas. plasmacytomas, plaques and tumors of mycosis fungoides and cutaneous T-cell lymphoma/leukemia; lymphomas including both Hodgkin's and non-Hodgkin's lymphomas; prophylaxis of autoimmune diseases including rheumatoid, immune and degenerative arthritis; ocular diseases including diabetic retinopathy, retinopathy of prematurity, corneal graft rejection, retrolental fibroplasia, neovascular glaucoma, rubeosis, retinal ncovascularization due to macular degeneration and hypoxia; abnormal neovascularization conditions of the eye; skin diseases including psoriasis; blood vessel diseases including hemagiomas and capillary proliferation within atherosclerotic plaques; Osler-Webber Syndrome; myocardial angiogenesis; plaque neovascularization; telangiectasia; hemophiliac joints; angiofibroma; wound granulation; diseases characterized by excessive or abnormal stimulation of endothelial cells including intestinal adhesions, Crohn's disease, atherosclerosis, scleroderma and hypertrophic scars (i.e. keloids) and diseases which have angiogenesis as a pathologic consequence including cat scratch disease (*Rochele minalia quintosa*) and ulcers (*Helicobacter pylori*). Another use is as a birth control agent which inhibits ovulation and establishment of the placenta.

The compounds of the present invention may also be useful for the prevention of metastases from the tumors described above either when used alone or in combination with radiotherapy and/or other chemotherapeutic treatments conventionally administered to patients for treating cancer. For example, when used in the treatment of solid tumors, compounds of the present invention may be administered with chemotherapeutic agents such as alpha inteferon, COMP (cyclophosphamide, vincristine, methotrexate and prednisone), etoposide, mBACOD (methortrexate, bleomycin, doxorubicin, cyclophosphamide, vincristine and dexamethasone), PRO-MACE/MOPP (prednisone, methotrexate (w/leucovin rescue), doxorubicin, cyclophosphamide, taxol, etoposide/mechlorethamine, vincristine, prednisone and procarbazine), vincristine, vinblastine, angioinhibins, TNP-470, pentosan polysulfate, platelet factor 4, angiostatin, LM-609, SU-101, CM-101. Techgalan, thalidomide, SP-PG and the like. Other chemotherapeutic agents include alkylating agents such as nitrogen mustards including mechloethamine, melphan, chlorambucil, cyclophosphamide and ifosfamide; nitrosoureas including carmustine, lomustine, semustine and streptozocin; alkyl sulfonates including busulfan; triazines including dacarbazine; ethyenimines including thiotepa and hexamethylmelamine; folic acid analogs including methotrexate: pyrimidine analogues including 5-fluorouracil, cytosine arabinoside; purine analogs including 6-mercaptopurine and 6-thioguanine; antitumor antibiotics including actinomycin D; the anthracyclines including doxorubicin, bleomycin, mitomycin C and methramycin; hormones and hormone antagonists including tamoxifen and cortiosteroids and miscellaneous agents including cisplatin and brequinar.

The compounds of the present invention may be used in the form of pharmaceutically acceptable salts derived from inorganic or organic acids. By "pharmaceutically acceptable salt" is meant those salts which are, within the scope of sound medical judgement, suitable for use in contact with the tissues of humans and lower animals without undue toxicity, irritation, allergic response and the like and are commensurate with a reasonable benefit/risk ratio. Pharmaceutically acceptable salts are well-known in the art. For example, S. M. Berge, *et al*. describe pharmaceutically acceptable salts in detail in *J. Pharmaceutical Sciences,* **1977,** 66: 1 *et seq*. The salts may be prepared in situ during the final isolation and purification of the compounds of the invention or separately by reacting a free base function with a suitable acid. Representative acid addition salts include, but are not limited to acetate, adipate, alginate, citrate, aspartate, benzoate, benzenesulfonate, bisulfate. butyrate, camphorate, camphorsufonate, digluconate, glycerophosphate, hemisulfate, heptanoate, hexanoate, fumarate, hydrochloride, hydrobromide, hydroiodide, 2-hydroxyethansulfonate (isethionate), lactate, maleate, methanesulfonate, nicotinate, 2-naphthalenesulfonate, oxalate, pamoate, pectinate, persulfate, 3-phenylpropionate, picrate, pivalate, propionate, succinate, tartrate, thiocyanate, phosphate, glutamate, bicarbonate, p-toluenesulfonate and undecanoate. Also, the basic nitrogen-containing groups can be quatemized with such agents as lower alkyl halides such as methyl, ethyl, propyl, and butyl chlorides, bromides and iodides; dialkyl sulfates like dimethyl, diethyl, dibutyl and diamyl sulfates; long chain halides such as decyl, lauryl, myristyl and stearyl chlorides, bromides and iodides; arylalkyl halides like benzyl and phenethyl bromides and others. Water or oil-soluble or dispersible products are thereby obtained. Examples of acids which may be employed to form pharmaceutically acceptable acid addition salts include such inorganic acids as hydrochloric acid, hydrohromic acid, sulphuric acid and phosphoric acid and such organic acids as oxalic acid, maleic acid, succinic acid and citric acid.

Basic addition salts can he prepared *in situ* during the final isolation and purification of compounds of this invention by reacting a carboxylic acid-containing moiety with a suitable base such as the hydroxide, carbonate or bicarbonate of a pharmaceutically acceptable metal cation or with ammonia or an organic primary, secondary or tertiary amine. Pharmaceutically acceptable salts include, but are not limited to, cations based on alkali metals or alkaline earth metals such as lithium, sodium, potassium, calcium, magnesium and aluminum salts and the like and nontoxic quaternary ammonia and amine cations including ammonium, tetramethylammonium, tetraethylammonium, methylamine, dimethylamine, trimethylamine, triethylamine, diethylamine, ethylamine and the like. Other representative organic amines useful for the formation of base addition salts include ethylenediamine, ethanolamine, diethanolamine, piperidine, piperazine and the like. Preferred salts of the compounds of the invention include phosphate, tris and acetate.

Compounds of this invention may be combined with pharmaceutically acceptable sustained-release matrices, such as biodegradable polymers, to form therapeutic pocompositions. A sustained-release matrix, as used herein, is a matrix made of materials, usually polymers, which are degradable by enzymatic or acid-base hydrolysis or by dissolution. Once inserted into the body, the matrix is acted upon by enzymes and body fluids. A sustained-release matrix is desirably chosen from biocompatible materials such as liposomes, polylactides (polylactic acid), polyglycolide (polymer of glycolic acid), polylactide co-glycolide (copolymers of lactic acid and glycolic acid) polyanhydrides, poly(ortho)esters, polypeptides, hyaluronic acid, collagen, chondroitin sulfate, carboxylic acids, fatty acids, phospholipids, polysaccharides, nucleic acids, polyamino acids, amino acids such as phenylalanine, tyrosine, isoleucine, polynucleotides, polyvinyl propylene, polyvinylpyrrolidone and silicone. A preferred biodegradable matrix is a matrix of one of either polylactide, polyglycolide, or polylactide co-glycolide (co-polymers of lactic acid and glycolic acid).

Compounds of this invention or combinations thereof may be combined with pharmaceutically acceptable excipients or carriers to form therapeutic compositions. A pharmaceutically acceptable carrier or excipient refers to a non-toxic solid, semi-solid or liquid filler, diluent, encapsulating material or formulation auxiliary of any type. The compositions may be administered parenterally, sublingually, incracisternally, intravaginally, intraperitoneally, rectally, bucally or topically (as by powder, ointment, drops. transdermal patch or iontophoresis device).

The term "parenteral," as used herein, refers to modes of administration which include intravenous, intramuscular, intraperitoneal, intrasternal, subcutaneous and intraarticular injection and infusion. Pharmaceutical compositions for parenteral injection comprise pharmaceutically acceptable sterile aqueous or nonaqueous solutions, dispersions, suspensions or emulsions as well as sterile powders for reconstitution into sterile injectable solutions or dispersions just prior to use. Examples of suitable aqueous and nonaqueous carriers, diluents, solvents or vehicles include water. ethanol, polyols (such as glycerol, propylene glycol, polyethylene glycol and the like), carboxymethylcellulose and suitable mixtures thereof, vegetable oils (such as olive oil) and injectable organic esters such as ethyl oleate. Proper fluidity may be maintained, for example, by the use of coating materials such as lecithin, by the maintenance of the required particle size in the case of dispersions and by the use of surfactants. These compositions may also contain adjuvants such as preservatives, wetting agents, emulsifying agents and dispersing agents. Prevention of the action of microorganisms may be ensured by the inclusion of various antibacterial and antifungal agents such as paraben, chlorobutanol, phenol, sorbic acid and the like. It may also be desirable to include isotonic agents such as sugars, sodium chloride and the like. Prolonged absorption of the injectable pharmaceutical form may be brought about by the inclusion of agents, such as aluminum monostearate and gelatin, which delay absorption. Injectable depot forms are made by forming microencapsule matrices of the drug in biodegradable polymers such as polylactide-polyglycolide, poly(orthoesters) and poly(anhydrides). Depending upon the ratio of drug to polymer and the nature of the particular polymer employed, the rate of drug release can be controlled. Depot injectable formulations are also prepared by entrapping the drug in liposomes or microemulsions which are compatible with body tissues. The injectable formulations may be sterilized, for example, by filtration through a bacterial-retaining filter or by incorporating sterilizing agents in the form of sterile solid compositions which can be dissolved or dispersed in sterile water or other sterile injectable media just prior to use.

Topical administration includes administration to the skin, mucosa and surfaces of the lung and eye. Compositions for topical administration, including those for inhalation, may be prepared as a dry powder which may be pressurized or non-pressurized. In non-pressurized powder compositions, the active ingredient in finely divided form may be used in admixture with a larger-sized pharmaceutically acceptable inert carrier comprising particles having a size, for example, of up to 100 micrometers in diameter. Suitable inert carriers include sugars such as lactose. Desirably, at least 95% by weight of the particles of the active ingredient have an effective particle size in the range of 0.01 to 10 micrometers. For topical administration to the eye, a compound of the invention is delivered in a pharmaceutically acceptable ophthalmic vehicle such that the compound is maintained in contact with the ocular surface for a sufficient time period to allow the compound to penetrate the corneal and internal regions of the eye, as, for example, the anterior chamber, posterior chamber, vitreous body, aqueous humor, vitreous humor, cornea, iris/cilary, lens, choroid/retina and sclera. The pharmaceutically acceptable ophthalmic vehicle may, for example, be an ointment, vegetable oil or an encapsulating material. Alternatively, a compound of the invention may be injected directly into the vitreous and aqueous humor.

The composition may be pressurized and contain a compressed gas such as nitrogen or a liquified gas propellant. The liquified propellant medium and indeed the total composition is preferably such that the active ingredient does not dissolve therein to any substantial extent. The pressurized composition may also contain a surface active agent such as a liquid or solid non-ionic surface active agent or may be a solid anionic surface active agent. It is preferred to use the solid anionic surface active agent in the form of a sodium salt.

Compositions for rectal or vaginal administration are preferably suppositories which may be prepared by mixing the compounds of this invention with suitable nonirritating excipients or carriers such as cocoa butter, polyethylene glycol or a suppository wax which are solids at room temperature but liquids at body temperature and therefore melt in the rectum or vaginal cavity and release the active compound.

Compounds of the present invention may also be administered in the form of liposomes. As is known in the art, liposomes are generally derived from phospholipids or other lipid substances. Liposomes are formed by mono- or multi-lamellar hydrated liquid crystals that are dispersed in an aqueous medium. Any non-toxic, physiologically acceptable and mecabolizable lipid capable of forming liposomes can be used. The present compositions in liposome form may contain, in addition to a compound of the present invention, stabilizers, preservatives, excipients and the like. The preferred lipids are the phospholipids and the phosphatidyl cholines (lecithins), both natural and synthetic. Methods to form liposomes are known in the art. See, for example, Prescott, Ed., *Methods in Cell Biology,* Volume XIV, Academic Press, New York, N.Y. (**1976**), p. 33 *et seq.*

When used in the above or other treatments, a therapeutically effective amount of one of the compounds of the present invention may be employed in pure form or, where such forms exist, in pharmaceutically acceptable salt form and with or without a pharmaceutically acceptable excipient. A "therapeutically effective amount" of the compound of the invention means a sufficient amount of the compound to treat an angiogenic disease (for example, to limit tumor growth or to slow or block tumor metastasis) at a reasonable benefit/risk ratio applicable to any medical treatment. It will be understood, however, that the total daily usage of the compounds and compositions of the present invention will be decided by the attending physician within the scope of sound medical judgment, The specific therapeutically effective dose level for any particular patient will depend upon a variety of factors including the disorder being treated and the severity of the disorder; activity of the specific compound employed; the specific composition employed; the age, body weight, general health, sex and diet of the patient; the time of administration; the route of administration; the rate of excretion of the specific compound employed; the duration of the treatment; drugs used in combination or coincidental with the specific compound employed and like factors well known in the medical arts. For example, it is well within the skill of the art to start doses of the compound at levels lower than those required to achieve the desired therapeutic effect and to gradually increase the dosage until the desired effect is achieved. Total daily dose of compounds of this invention to be administered locally or systemically to a human or other mammal host in single or divided doses may be in amounts, for example, from 0.01 to 200 mg/kg body weight daily and more usually 1 to 300 mg/kg body weight. If desired, the effective daily dose may be divided into multiple doses for purposes of administration. Consequently, single dose compositions may contain such amounts or submultiples thereof to make up the daily dose.

It will be understood that agents which can be combined with the compound of the present invention for the inhibition, treatment or prophylaxis of angiogenic diseases are not limited to those listed above, but include, in principle, any agents useful for the treatment or prophylaxis of angiogenic diseases.

### Preparation of Compounds of the Invention

### Abbreviations

Abbreviations which have been used in the descriptions of the scheme and the examples that follow are: DMSO for dimethylsulfoxide, DME for dimethoxyethane, EtOAc for ethyl acetate, and THF for tetrahydrofuran.

### Synthetic Methods

The compounds and processes of the present invention will be better understood in connection with the following synthetic schemes which illustrate the methods by which the compounds of the invention may be prepared.

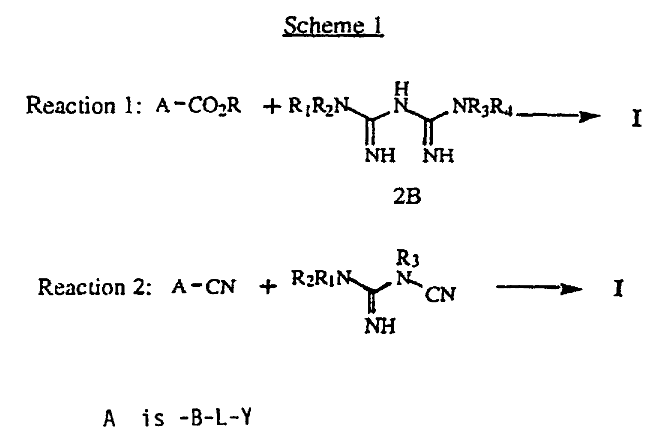

As shown in Scheme 1, the triazine ring of the compounds of Formula **I** were prepared from condensation of esters with biguanide (Reaction 1) or from condensation of nitriles and cyanoguanidine (Reaction 2). Reaction 2 was performed in a polar, high boiling solvent such as 2-methoxyethanol and in the presence of a strong base such as potassium hydroxide. Reaction 1 was performed in an an alcohol, preferably methanol. The ester and nitrile precursors were purchased from commercial sources or prepared using known chemical transformations.

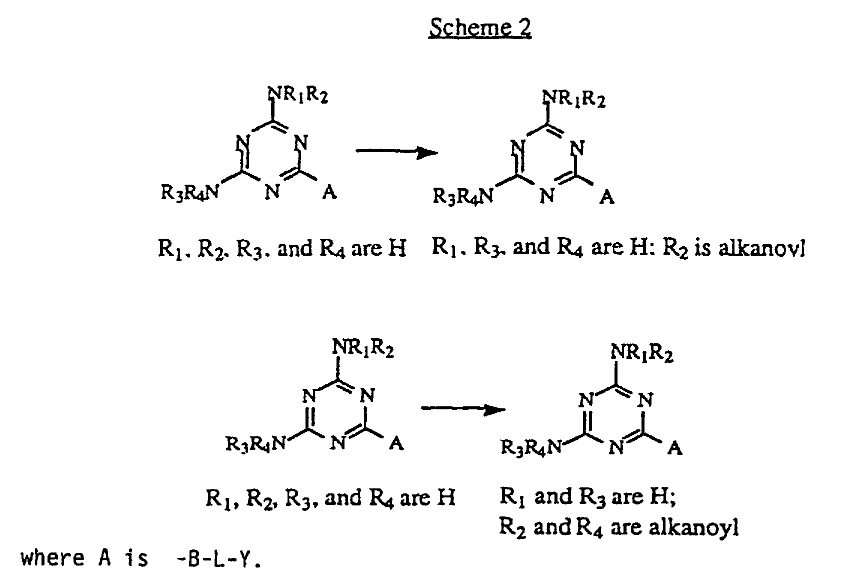

As shown in Scheme 2, selective mono acylation to provide compounds of Formula **I** was accomplished by heating a diaminotriazine precursor with a carboxylic acid anhydride at elevated temperature, preferably 80-90 °C. Alternatively, 2,4-diacylation was accomplished by heating the diaminotriazine precursor with a carboxylic acid anhydride at higher temperatures, preferably 140-160 °C.

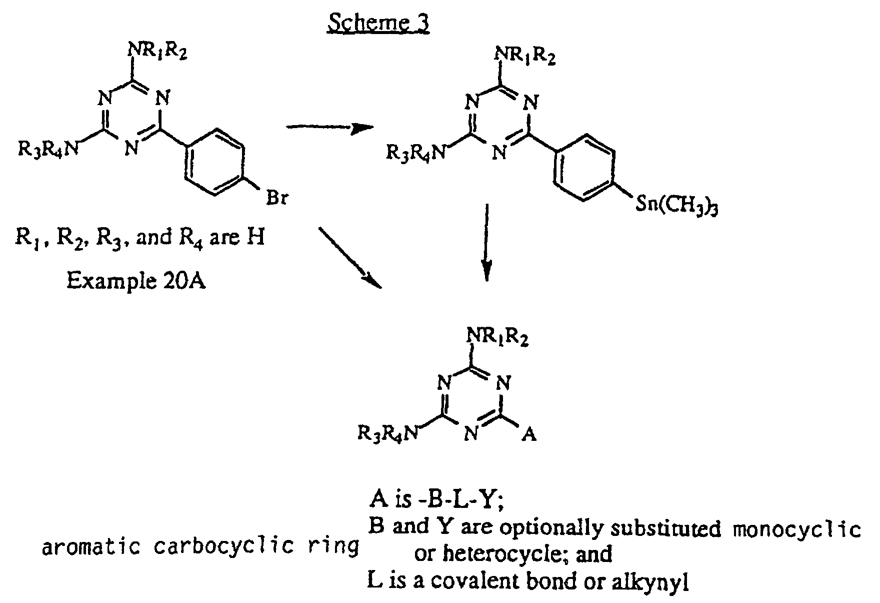

As shown in Scheme 3, 2,4-diamino-6-bromoaryl-triazines were converted to compounds of Formula **I** using transition metal-catalyzed cross-coupling reactions catalyzed by palladium catalysts such as tetrakis(triphenylphosphine) palladium. Also, conversion of Example 20A to a 2,4-diamino-6-(trialkylstannyl)aryl-triazine by treatment with organotin reagents, preferably hexamethylditin, in the presence of a palladium catalyst such as tetrakis(triphenylphosphine) palladium, followed by cross-coupling with aryl bromides, provided an alternative route to compounds of Formula **I.** Treatment of Example 20A with ethynyltin reagents such as trimethyl(phenylethylyl)tin in the presence of palladium catalysts such as tetrakis(triphenylphosphine) palladium also provided compounds of Formula **I.**

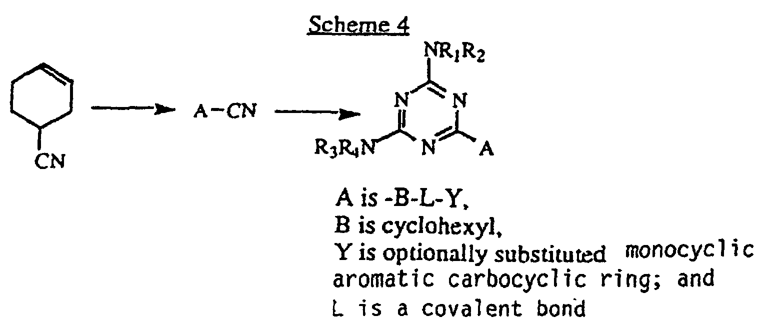

As shown in Scheme 4, compounds of Formula **I** were prepared by Friedel Crafts alkylation of aryl groups with a cycloalkenyl nitrile followed by elaboration of the nitrile intermediate as described in Scheme 1 (Reaction 2).

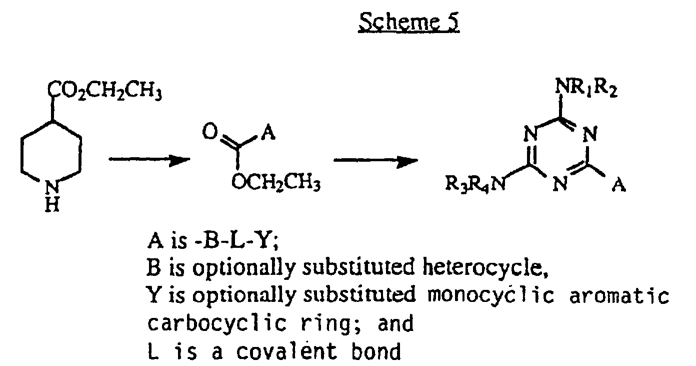

As shown in Scheme 5, piperidinyl aryl esters were converted to compounds of Formula **I** by arylation of isonipecotic acid esters with triarylbismuth reagents in the presence of copper (II) acylates.

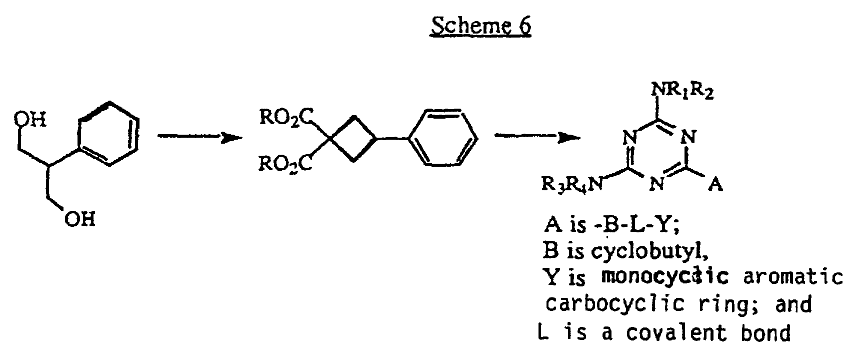

As shown in Scheme 6, compounds of Formula **I** were prepared by condensation of bis-tosylates with malonic esters to construct cycloalkane rings, mono-decarboxylated at elevated temperatures, and further processed according to Scheme 1 (Reaction 1).

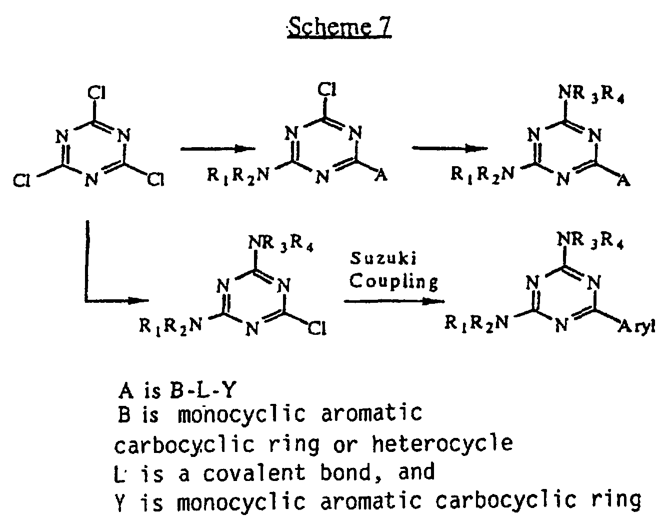

As shown in Scheme 7, diaminotriazines bearing alkyl substituents on the amino groups can be prepared in a controlled and predicable manner by sequential displacement of chlorines from the triazine ring. The 6-aryl or heteroaryl substituent may be introduced first by nucleophilic addition, for example as a Grignard reagent to cyanuric chloride, or after nitrogen introduction, for example by Pd-catalyzed Suzuki cross coupling with a boronic acid.

The compounds of the present invention and the above processes will be better understood in connection with the following examples, which are intended as an illustration of and not a limitation upon the scope of the invention. The compounds of Examples 5, 11, 13, 16, 23, 25, 26, 35, 38, 45, 47-56, 59, 60, 65 and 68-72 are not compounds of the present invention.

### Example 1

### 6-[1-(diphenylmethyl)-3-azetidinyl]-1,3,5-triazine-2,4-diamine

A solution of 1-(diphenylmethyl)-3-azetidinecarbonitrile (500 mg, 2.01 mmol), dicyandiamide (220 mg, 2.62 mmol) and KOH (34 mg, 0.604 mmol) in 2-methoxyethanol (10 mL) was heated at reflux for 4 hours, diluted with water, and cooled to room temperature. The precipitate was rinsed with water and dried under vacuum to provide the title compound.
mp 126-128 °C;
MS (DCI/NH₃) m/e 333 (M+H)⁺;
¹H NMR (300 MHz, DMSO-d₆) δ 7.3 (d, 4H), 7.2 (t, 4H), 7.05 (t, 2H), 6.5-6.7 (br s, 4H), 4.35 (s, 1H), 3.2-3.3 (m, 3H), 3.1-3.15 (m, 2H);
Anal. calcd for C₁₉H₂₀N₆·0.75H₂O: C, 65.97; H, 6.26; N, 24.29. Found: C, 65.67; H, 5.65; N, 23.84.

### Example 2

### 6-(1-phenyl-4-piperidinyl)-1,3,5-triazine-2,4-diamine

### Example 2A

A solution of triphenylbismuth (5.02 g, 11.4 mmol), cupric acetate (1.79 g, 9.85 mmol), and ethyl isonipecotate (1.5 mL, 9.7 mmol) in dichloromethane (100 mL) was stirred at room temperature for 18 hours, diluted with water, and filtered through Celite®. The organic layer was dried (MgSO₄), and concentrated. The residue was purified by flash chromatography on silica gel with 0-2% acetone/dichloromethane to provide the designated compound.
MS (DCI/NH₃) m/e 234 (M+H)⁺.

### Example 2B

The designated compound was prepared as in Inorganic Synthesis, Volume 7, pp. 56-58 (1963).

### Example 2C

### 6-(1-Phenyl-4-piperidinyl)-1,3,5-triazine-2,4-diamine

A solution of Examples 2A (0.464 g, 1.99 mmol) and 2B (0.211 g, 2.09 mmol) in methanol (4 mL) was stirred at room temperature for 16 hours. The precipitate was rinsed with methanol, dried under vacuum, and recrystallized from dioxane/ethanol to provide the title compound.
mp 202-204 °C;
MS (DCI/NH₃) m/e 271 (M+H)⁺;
¹H NMR (300 MHz, DMSO-d₆) δ 7.19 (t, 2H), 6.94 (d, 2H), 6.72 (t, 1H), 6.56 (br s, 4H), 4.11 (q, 2H), 3.77 (m, 2H), 2.75 (dt, 2H), 2.41 (m, 1H), 1.79 (m, 2H);
Anal. calcd for C₁₄H₁₈N₆·0.67H₂O: C, 59.58; H, 6.90; N, 27.78, Found: C, 59.27; H, 6.79; N, 25.51.

### Example 3

### trans-6-(4-phenylcyclohexyl)-1,3,5-triazine-2,4-diamine

### Example 3A

### 4-phenylhexenecarbonitrile

A solution of cyclohexenecarbonitrile (9 mL, 80.6 mmol) and benzene (75 mL) was treated portionwise with AlCl₃ (13 g, 97 mmol) then stirred at room temperature for 2 hours. The mixture was poured onto ice and extracted with ethyl acetate. The extract was washed sequentially with water and brine, dried (MgSO₄), and concentrated. The residue was distilled at 125 °C (0.6 mm Hg) to provide the title compound.
MS (DCI/NH₃) m/e 203 (M+NH₄)⁺.

### Example 3B

### trans-6-(4-phenylcyclohexyl)-1,3,5-triazine-2,4-diamine

Example 3A was processed as in Example 1 to provide the title compound. MS (DCI/NH₃) m/e 270 (M+H)⁺;
¹H NMR (300 MHz, DMSO-d₆) δ 7.20-7.32 (m, 4H), 7.12-7.18 (m, 1H), 6.57 (br s, 4H), 2.46 (tt, 1H), 2.32 (tt, 1H), 1.80-1.93 (m, 4H), 1.41-1.66 (m, 4H);
Anal. calcd for C₁₅H₁₉N₅: C, 66.88; H, 7.11; N, 26.00. Found: C, 66.85; H, 7.00; N, 26.08.

### Example 4

### 6-[3-(1H-pyrrol-1-yl)phenyl]-1,3,5-triazine-2,4-diamine

3-(1H-pyrrol-1-yl)benzonitrile was processed as in Example 1 to provide the title compound.
mp 164-170 °C;
MS (DCI/NH₃) m/e 253 (M+H)⁺;
¹H NMR (300 MHz, DMSO-d₆) δ 8.35 (s, 1H), 8.15 (d, 1H), 7.7 (dd, 1H), 7.6-7.5 (m, 1H), 7.3 (t, 3H), 7.0-6.8 (br s, 4H), 6.3-6.25 (m, 2H);
Anal. calcd for C₁₃H₁₂N₆: C, 61.89; H, 4.79; N, 33.31. Found: C, 62.20; H, 4.56; N, 32.39.

### Example 5

### cis/trans-6-(3-phenylcyclobutyl)-1,3,5-triazine-2,4-diamine

A solution of cis/trans-methyl 3-phenylcyclobutane-1-carboxylate, prepared as in J. Am. Chem. Soc. 1985, 107, 7247-7257, was processed as in Example 2C to provide the title compounds.
mp 98-102 °C;
¹H NMR (300 MHz, DMSO-d₆) δ 7.31 (m, 4H), 7.19 (m, 1H), 6.60 (m, 4H), 3.62 (m, 0.4H), 3.43 (m, 0.4H), 3.18 (m, 0.8H), 2.88 (m, 0.8H), 2.56 (m, 1.2H), 2.38 (m, 2.4H); Anal. calcd for C₁₃H₁₅N₅·0.5CH₃CO₂CH₂CH₃: C, 63.14; H, 6.71; N, 24.54. Found: C, 62.75; H, 6.73; N, 24.48.

### Example 6

### 6-[1,1'-biphenyl]-2-yl-1,3,5-triazine-2,4-diamine

[1,1'-biphenyl]-2-carbonitrile was processed as in Example 1 to provide the title compound.
MS (DCI/NH₃) m/e 264 (M+H)⁺;
¹H NMR (300 MHz, DMSO-d₆) δ 7.63-7.2 (m, 5H), 7.37-7.27 (m, 4H), 6.6 (br s, 4H);
Anal. calcd for C₁₅H₁₃N₅: C, 68.42; H, 4.97; N, 26.59. Found: C, 67.85; H, 4.94 ; N, 26.50.

### Example 7

### 6-(4'-nitro[1,1'-biphenyl]-4-yl)-1,3,5-triazine-2,4-diamine

4'-Nitro-[1,1'-biphenyl]-4-carbonitrile was processed as in Example 1 to provide the title compound.
mp >250 °C;
MS (DCI/NH₃) m/e 309 (M+H)⁺;
¹H NMR (300 MHz, DMSO-d₆) δ 8.5-8.4 (m, 4H), 8.1 (d, 2H), 7.95 (d, 2H), 6.85 (br s, 4H);
Anal. calcd for C₁₅H₁₂N₆O₂: C, 58.43; H, 3.92; N, 27.42. Found: C, 58.46; H, 3.76; N, 27.12.

### Example 8

### trans-6-[4-(4-pentylcyclohexyl)phenyl]-1,3,5-triazine-2,4-diamine

4-(Trans-4-pentylcyclohexyl)benzonitrile was processed as in Example 1 to provide the title compound.
mp >250 °C;
MS (DCI/NH₃) m/e 340 (M+H)⁺;
¹H NMR (300 MHz, DMSO-d₆) δ 8.2 (d, 2H), 7.3 (d, 2H), 6.75 (bs, 4H), 1.85 (d, 4H), 1.55-1.4 (m, 2H), 1.37-1.2 (m, 10H), 1.1-1.05 (m, 2H), .85 (t, 3H);
Anal. calcd for C₂₀H₂₉N₅: C, 70.76; H, 8.61; N, 20.62. Found: C, 70.71; H. 8.73; N, 20.67.

### Example 9

### 6-(4-phenoxyphenyl)-1,3,5-triazine-2,4-diamine

4-phenoxybenzonitrile was processed as in Example 1 to provide the tide compound.
mp 198-200 °C;
MS (DCI/NH₃) m/e 280 (M+H)⁺;
¹H NMR (300 MHz, DMSO-d₆) δ 8.3-8.2 (m, 2H), 7.5-7.4 (m, 2H), 7.2 (t, 1H), 7.17-7.0 (m, 4H), 6.9-6.65 (br s, 4H);
Anal. calcd for C₁₅H₁₃N₅O: C, 64.51; H, 4.69; N, 25.07. Found: C, 63.84; H, 4.67; N, 24.90.

### Example 10

### N-cyclohexyl-N'-[4-(4,6-diamino-1,3,5-triazin-2-yl)phenyl]urea

### Example 10A

4-Aminobenzonitrile was processed as in Example 1 to provide the designated compound.
MS (DCI/NH₃) m/e 203 (M+H)⁺.

### Example 10B

### N-cyclohexyl-N'-[4-(4,6-diamino-1,3,5-triazin-2-yl)phenyl]urea

A mixture of Example 10A (1.0 g; 4.9 mmol), cyclohexylisocyanate (610 mg, 4.9 mmol), and triethylamine (0.68 mL, 4.9 mmol) in dioxane was stirred overnight at room temperature. The precipitate was washed with water and dried under vacuum to provide the title compound.
MS (DCI/NH₃) m/e 328 (M+H)⁺;
¹H NMR (300 MHz, DMSO-d₆) δ 8.4 (s, 1H), 8.23 (t, 1H), 8.8-8.75 (m, 1H), 7.55-7.45 (m, 1H), 7.25-7.2 (t, 1H), 7.0-6.8 (hr s, 4H), 6.0 (d, 1H), 3.55-3.4 (m, 1H), 1.9-1.8 (m, 2H), 1.7-1.6 (m, 2H), 1.59-1.5 (m, 1H), 1.2-0.5 (m, 5H);
Anal. calcd for C₁₅H₂₁N₇O: C, 58.70; H, 6.47; N, 29.95. Found: C, 58.49; H, 6.59; N, 29.49.

### Example 11

### (4,6-diamino-1,3,5-triazine-2-yl)phenylmethenone

4-Cyanobenzophenone was processed as in Example 1 to provide the title compound.
mp >250 °C;
¹H NMR (300 MHz, DMSO-d₆) δ 8.4 (d, 2H), 7.9 (d, 2H), 7.8 (m, 2H), 7.7 (m, 1H), 7.6 (t, 2H), 6.9 (br s, 4H);
MS (DCI/NH₃) m/e 292 (M+H)⁺;
Anal. calcd for C₁₆H₁₃N₅O: C, 65.97; H, 4.50; N, 24.04. Found: C, 65.74; H, 4.32; N, 23.93.

### Example 12

### N-[4-(4,6-diamino-1,3,5-triazin-2-yl)phenyl]-N'-phenyl urea

Example 10A was processed as in Example 10B but substituting phenylisocyanate for cyclohexylisocyanate to provide the title compound.
¹H NMR (300 MHz, DMSO-d₆) δ 8.8 (s, 1H), 8.65 (s, 1H), 8.35 (t, 1H), 7.9 (d, 1H), 7.6-7.5 (m, 1H), 7.49-7.44 (m, 2H), 7.35 (t, 1H), 7.29 (t, 2H), 7.0 (t, 1H), 6.8-6.7 ( br s, 4H);
Anal. calcd for C₁₆H₁₅N₇O: C, 59.80; H, 4.70; N, 30.51. Found: C, 59.61; H, 4.72; N, 29.91.

### Example 13

### 6-(1,4-dioxa-8-azaspiro[4,5]dec-8-yl)-1,3,5-triazine-2,4-diamine

A mixture of 2,4-diamino-6-chloro-1,3,5-triazine (2 g, 14 mmol), 1,4-dioxa-8-azaspiro[4.5]decane (3 g, 21 mmol), and KOH (100 mg, 1.8 mmol) in dioxane (10 mL) and ethanol (40 mL) was heated at reflux overnight, diluted with water, and filtered. The precipitate was rinsed with water and dried under vacuum to provide the title compound. mp 209-211 °C;
MS (DCI/NH₃) m/e 253 (M+H)⁺;
¹H NMR (300 MHz, DMSO-d₆) δ 6.14 (br s, 4H), 3.90 (s, 4H), 3.75-3.68 (m, 4H), 1.58-1.51 (m, 4H);
Anal. calcd for C₁₀H₁₆N₆O₂: C, 47.61; H, 6.39; N, 33.31. Found: C, 47.45; H, 6.34; N, 33.24.

### Example 14

### 6-(4'-pentyl[1,1'-biphenyl]-4-yl)-1,3,5-triazine-2,4-diamine

4'-Pentyl[1,1'-biphenyl]-4-carbonitrile was processed as in Example 1 to provide the title compound.
mp 242-244 °C;
MS (DCI/NH₃) m/e 334 (M+H)⁺;
¹H NMR (300 MHz, DMSO-d₆) δ 8.3 (d, 2H), 8.75 (d, 2H), 8.65 (d, 2H), 7.3 (d. 2H), 6.75-6.82 (br s, 4H), 2.6 (t, 2H), 1.6-1.7 (m, 2H), 1.3-1.4 (m, 4H), 0.95 (t, 3H);
Anal, calcd for C₂₀H₂₃N₅·0.25H₂O: C, 71.61; H, 7.09; N, 20.03. Found: C, 71.80; H, 7.00; N, 20.45.

### Example 15

### 6-[4'-pentyloxy[1,1'-biphenyl]-4-yl]-1,3,5-triazine-2,4-diamine

4'-(Pentyloxy)[1,1'-biphenyl]-4-carbonitrile was processed as in Example 1 to provide the title compound.
mp 246-249 °C;
MS (DCI/NH₃) m/e 350 (M+H)⁺;
¹H NMR (300 MHz, DMSO-d₆) δ 8.3 (d, 2H), 7.75-7.65 (m, 4H), 7.07 (d, 2H), 6.85-6.7 (br s, 4H), 4.05 (t, 2H), 1.8-1.7 (m, 2H), 1.5-1.3 (m, 4H), 0.9 (t, 3H);
Anal. calcd for C₂₀H₂₃N₅O: C, 68.75; H, 6.63; N, 20.04. Found: C, 68.64; H, 6.77; N, 19.94.

### Example 16

### 6-(6-methoxy-2-benzothiazolyl)-1,3,5-triazine-2,4-diamine

6-Methoxy-2-benzothiazolecarbonitrile was processed as in Example 1 to provide the title compound.
mp >250 °C;
MS (DCI/NH₃) m/e 275 (M+H)⁺;
¹H NMR (300 MHz, DMSO-d₆) δ 7.98 (d, 1H), 7.71 (d, 1H), 7.17 (dd, 1H), 7.16 (br s, 2H), 6.95 (br s, 2H), 3.85 (s, 3H);
Anal. calcd for C₁₁H₁₀N₆OS: C, 48.17; H, 3.67; N, 30.64. Found: C, 48.07; H, 3.75; N, 30.72.

### Example 17

### 6-(4'-amino[1,1'-biphenyl]-4-yl)-1,3,5-triazine-2,4-diamine

4'-Amino[1,1'-biphenyl]-4-carbonitrile was processed as in Example 1 to provide the title compound.
mp >250 °C;
MS (DCI/NH₃) m/e 279 (M+H)⁺;
¹H NMR (300 MHz, DMSO-d₆) δ 8.25 (d, 2H), 7.65 (d, 2H), 7.45 (d, 2H), 6.8-6.6 (m, 6H), 5.3 (s, 2H);
Anal. calcd for C₁₅H₁₄N₆: C. 64.73; H, 5.07; N, 30.20. Found: C, 64.34: H, 5.18; N. 29.91.

### Example 18

### 6-[4-(5-oxazolyl)phenyl]-1,3,5-triazine-2,4-diamine

4-(5-Oxazolyl)henzonitrile was processed as in Example 1 to provide the title compound.
MS (DCI/NH₃) m/e 255 (M+H)⁺;
¹H NMR (300 MHz, DMSO-d₆) δ 8.55 (s, 1H), 8.37 (d, 2H), 7.9-7.8 (t, 3H), 6.9-6.7 (br s, 4H);
Anal. calcd for C₁₂H₁₀N₆O: C, 56.69; H, 3.96; N, 33.05. Found: C, 56.40; H, 4.02; N, 33.11.

### Example 19

### 6-[4-[[5-(trifluoromethyl)-2-pyridinyl]oxy]phenyl]-1,3,5-triazine-2,4-diamine

4-[[5-(Trifluoromethyl)-2-pyridinyl]oxy]benzonitrile was processed as in Example 1 to provide the title compound.
MS (DCI/NH₃) m/e 349 (M+H)⁺;
¹H NMR (300 MHz, DMSO-d₆) δ 8.6 (s, 1H), 8.6-8.5 (m, 3H), 7.4-7.3 (m, 3H), 6.9-6.7 (br s, 4H);
Anal. calcd for C₁₅H₁₁F₃N₆O: C, 51.73; H, 3.18; N, 24.13. Found: C, 51.67; H, 3.20; N, 23.83.

### Example 20

### 4'-(4,6-diamino-1,3,5-triazine-2-yl)[1,1'-biphenyl]-4-carbonitrile

### Example 20A

4-Bromohenzonitrile was processed as in Example 1 to provide the designated compound.
MS (DCI/NH₃) m/e 267 (M+H)⁺.

### Example 20B

A solution of Example 20A (0.76 g, 2.9 mmol) and tetrakis(triphenylphosphine) palladium (0.17 g, 0.15 mmol) in dry, degassed dimethylacetamide (45 mL) was heated to 100 °C, treated with hexamethylditin (1.0 g 3.1 mmol), heated at 100 °C for 3 hours, treated with ethyl acetate, washed sequentially with 1M NaOH and brine, dried (MgSO₄), and concentrated to provide the designated compound.
MS (DCI/NH₃) m/e 352 (M+H)⁺.

### Example 20C

### 4'-(4,6-diamino-1,3,5-triazine-2-yl)[1,1'-biphenyl]-4-carbonitrile

A solution of Example 20B (0.95 g, 2.7 mmol), 4-bromobenzonitrile (0.55 g, 3.0 mmol) and tetrakis(triphenylphosphine) palladium (0.20 g, 0.17 mmol) in dry, degassed dimethylacetamide (45 mL) was heated at 100 °C for 3 hours, cooled to room temperature, treated with ethyl acetate, washed sequentially with 1M NaOH and brine, dried (MgSO₄), and concentrated. The residue was recrystallized from dioxane/ethanol to provide the title compound.
mp >260 °C;
MS (DCI/NH₃) m/e 289 (M+H)⁺;
¹H NMR (300 MHz, DMSO-d₆) δ 8.36 (d, 2H), 7.96 (s, 4H), 7.88 (d, 2H), 6.81 (br s, 4H);
Anal. calcd for C₁₆H₁₂N₆·0.75H₂O: C, 63.67; H, 4.51; N, 27.84. Found: C, 64.06; H, 4.38; N, 27.17.

### Example 21

### 6-(4'-methoxy[1,1'-biphenyl]-4-yl)-1,3,5-triazine-2,4-diamine

A solution of Example 20A (0.749 g, 2.8 mmol) and tetrakis(triphenylphosphine) palladium (0.15 g, 0.13 mmol) in dry, degassed dimethylacetamide (45 mL) was heated to 100 °C, treated sequentially with 4-methoxyphenyl boronic acid (0.648 g, 4.3 mmol) in absolute ethanol (15 mL) and saturated NaHCO₃ (30 mL), heated at 100 °C for 3 hours, cooled to room temperature, treated with ethyl acetate, washed with brine, dried (MgSO₄), and concentrated. The residue was recrystallized from dioxane/ethanol to provide the title compound.
mp >260 °C;
MS (DCI/NH₃) m/e 294 (M+H)⁺;
¹H NMR (300 MHz, DMSO-d₆) δ 8.31 (d, 2H), 7.72 (t, 4H), 7.03 (d, 2H), 6.86 (br s, 4H), 3.81 (s, 3H);
Anal. calcd for C₁₆H₁₅N₅O·0.33H₂O: C, 64.21; H, 5.27; N, 23.40. Found: C, 64.26; H, 5.35; N, 23.43.

### Example 22

### 6-(4'-fluoro[1,1'-biphenyl]-4-yl)-1,3,5-triazine-2,4-diamine

Example 20A and 4-fluorophenyl boronic acid were processed as in Example 24 to provide the title compound.
mp >260 °C;
MS (DCI/NH₃) m/e 282 (M+H)⁺;
¹H NMR (300 MHz, DMSO-d₆) δ 8.32 (d, 2H), 7.77 (m. 4H), 7.32 (t. 2H), 6.75 (br s, 4H);
Anal. calcd for C₁₅H₁₂FN₅·0.25H₂O: C, 63.04; H, 4.41; N, 24.50. Found: C, 63.41; H, 4.49; N, 24.17.

### Example 23

### N-[4-(4,6-diamino-1,3,5-triazin-2-yl)phenyl]benzenesulfonamide

A solution of Example 10A, (575 mg, 2.8 mmol) and benzenesulfonyl chloride (554 mg, 3.1 mmol) in pyridine (5 mL) was heated at reflux for 4 hours, stirred overnight at room temperature, treated with water and extracted with ethyl acetate. The extract was washed with water and brine, dried (MgSO₄), and concentrated. The residue was recrystallized from ethanol to provide the title compound.
mp 197-199 °C;
MS (DCI/NH₃) m/e 343 (M+H)⁺;
¹H NMR (300 MHz, DMSO-d₆) δ 10.20 (br s, 1H), 8.03-8.01 (m, 1H), 7.94-7.91 (m, 1H), 7.80-7.78 (m, 2H), 7.60-7.50 (m, 3H), 7.34-7.25 (m, 1H), 7.22-7.19 (m, 1H);
Anal. calcd for C₁₅H₁₄N₆O₂S·C₂H₅OH: C, 52.56; H, 5.18; N, 21.63. Found: C, 52.47; H, 5.24; N, 21.54.

### Example 24

### 6-[1-([1,1'-biphenyl]-4-yl)-4-piperidinyl]-1,3,5-triazine-2,4-diamine

### Example 24A

A mixture of 4-bromobiphenyl (19.16 g, 82 mmol) in THF (820 mL) at -78 °C was treated with tert-butyllithium (100 mL of a 1.7 M solution in pentane, 170 mmol), stirred for 8 minutes, treated with bismuth trichloride (8.62 g, 27.4 mmol) in THF (100 mL), stirred an additional 3 hours, treated with saturated aqueous NH₄Cl, and extracted with ethyl acetate. The extract was washed with water and brine, dried over (MgSO₄) and concentrated. The residue was dried in a vacuum oven to provide the designated compound.
¹³C NMR (300 MHz, CDCl₃) δ 153.83, 141.04, 140.69, 138.07, 129.21, 128.75, 127.33, 127.07.

### Example 24B

### 6-[1-([1,1'-biphenyl]-4-yl)-4-piperidinyl]-1,3,5-triazine-2,4-diamine

Example 24A and ethyl isonipecotate were processed as in Examples 2A and 2C to provide the title compound.
mp >260 °C;
MS (DCI/NH₃) m/e 347 (M+H)⁺;
¹H NMR (300 MHz, DMSO-d₆) δ 7.58 (m, 2H), 7.47 (d, 2H), 7.39 (m, 2H), 7.23 (m, 1H), 6.97 (d, 2H), 6.59 (br s, 4H), 3.61 (m, 1H), 1.78 (m, 4H), 1.58 (m, 4H).

### Example 25

### N-[4-(4,6-diamino-1,3,5-triazin-2-yl)phenyl]-2-naphthalenesulfonamide

6-(4-Aminophenyl)-1,3,5-triazine-2,4-diamine was processed as in Example 23 but substituting 2-naphthalenesulfonyl chloride for benzenesulfonyl chloride to provide the title compound.
mp 230-233 °C;
MS (DCI/NH₃) m/e 393 (M+H)⁺;
¹H NMR (300 MHz, DMSO-d₆) δ 10.55 (s, 1H), 8.5 (s, 1H), 8.2-8.05 (m, 3H), 8.0 (d, 1H), 7.9-7.85 (m, 1H), 7.8-7.75 (m, 1H), 7.74-7.6 (m, 2H), 7.3-7.2 (m, 2H), 6.9-6.65 (br s, 4H);
Anal. calcd for C₁₉H₁₆N₆O₂S·1.5 C₄H₈O₂: C, 57.23; H, 5.37; N, 16.02. Found: C, 57.11; H, 5.33; N, 16.28.

### Example 26

### 2,5-dichloro-N-[4-(4,6-diamino-1,3,5-triazin-2-yl)phenyl]benzenesulfonamide

Example 10A was processed as in Example 23 but substituting 2,5-dichlorobenzenesulfonyl chloride for benzenesulfonyl chloride to provide the title compound.
mp 230-233 °C;
MS (DCI/NH₃) m/e 411 (M+H)⁺;
¹H NMR (300 MHz, DMSO-d₆) δ10.5 (s, 1H), 8.05 (m, 3H), 7.75-7.7 (m, 2H), 7.35 (t, 1H), 7.25-7.2 (m, 1H), 6.8-6.7 (br s, 4H);
Anal. calcd for C₁₅H₁₂Cl₂N₆O₂S·0.5CH₃CH₂OH C, 44.24; H, 3.48; N, 19.35. Found: C, 44.43; H. 3.26; N, 19.44.

### Example 27

### 6-(1-phenylcyclohexyl)-1,3,5-triazine-2,4-diamine

1-Phenylcyclohexanecarbonitrile was processed as in Example 1 to provide the title compound.
mp 153-155 °C;
MS (DCI/NH₃) m/e 270 (M+H)⁺;
¹H NMR (300 MHz, DMSO-d₆) δ 7.4-7.3 (m, 2H), 7.15 (t, 2H), 7.2-7.1 (m, 1H), 6.6-6.5 (br s, 4H), 2.7-2.6 (m, 2H), 1.75-1.6 (m, 2H), 1.6-1.2 (m, 6H);
Anal. calcd for C₁₅H₁₉N₅: C, 66.89; H, 7.11; N, 26.00. Found: C, 66.94; H, 7.20; N, 26.04.

### Example 28

### 6-[1-(4-methoxyphenyl)-4-piperidinyl]-1,3,5-triazine-2,4-diamine

Tris(4'-methoxy[1,1'-biphenyl]bismuth, prepared as in Example 24A, and ethyl isonipecotate were processed as in Examples 2A and 2C to provide the title compound. mp 204-205 °C;
MS (DCI/NH₃) m/e 301 (M+H)⁺;
¹H NMR (300 MHz, DMSO-d₆) δ 6.92 (d, 2H), 6.81 (d, 2H), 6.58 (m, 4H), 3.59 (m, 2H), 2.62 (m, 2H), 2.35 (m, 1H), 1.82 (m, 4H).

### Example 29

### 6-[2-[4-(trifluoromethyl)phenyl]-4-thiazolyl]-1,3,5-triazine-2,4-diamine

Ethyl 2-[4-(trifluoromethyl)phenyl]thiazole-4-carboxylate and Example 2B were processed as in Example 2C to provide the title compound.
mp >260 °C;
MS (DCI/NH₃) m/e 339 (M+H)⁺;
¹H NMR (300 MHz, DMSO-d₆) δ 8.42 (s, 1H), 8.19 (d, 2H), 7.91 (d, 2H), 6.82 (br s, 4H);
Anal. calcd for C₁₃H₉F₃N₆S: C, 46.15; H, 2.68; N, 24.84. Found: C, 45.85; H, 2.64; N, 24.44.

### Example 30

### 6-[1-(4-methoxyphenyl)cyclohexyl]-1,3,5-triazine-2,4-diamine

1-(4-Methoxyphenyl)cyclohexanecarbonitrile was processed as in Example 1 to provide the title compound.
mp 159-163 °C;
MS (DCI/NH₃) m/e 300 (M+H)⁺;
¹H NMR (300 MHz, DMSO-d₆) δ 7.15 (d, 2H), 6.8 (d, 2H), 6.6 (br s, 4H), 3.7 (s, 3H), 2.7-2.6 (m, 2H), 1.7-1.6 (m, 2H), 1.6-1.2 (m, 6H);
Anal. calcd for C₁₆H₂₁N₅O: C, 64.19; H, 7.07; N, 23.39. Found: C, 64.13; H, 7.07; N, 23.25.

### Example 31

### 6-[4-(2-thienyl)phenyl]-1,3,5-triazine-2,4-diamine

A solution of Example 20A (500 mg, 1.9 mmol) and 2-tri-n-butyltinthiophene (840 mg, 2.2 mmol) in dry, degassed dimethylacetamide (15 mL) was treated with tetrakis(triphenylphosphine) palladium (115 mg, 0.1 mmol), heated at 100 °C for 3 hours, cooled, treated with 1N NaOH, and extracted with ethyl acetate. The extract was washed with brine, dried (MgSO₄), and concentrated. The residue was recrystallized from ethanol/dioxane to provide the title compound.
mp >260;
MS (DCI/NH₃) m/e 270 (M+H)⁺;
¹H NMR (300 MHz, DMSO-d₆) δ 8.31-8.24 (m, 2H), 7.8-7.72 (m, 2H), 7.62-7.59 (m, 2H), 7.2-7.16 (m, 1H), 6.92 (br s, 4H);
Anal. calcd for C₁₃H₁₁N₅S: C, 57.97; H, 4.11; N, 26.00. Found: C, 57.91; H, 4.06; N, 25.83.

### Example 32

### 6-[4-(phenylethynyl)phenyl]-1,3,5-triazine-2,4-diamine

### Example 32A

4-Bromobenzonitrile and trimethyl(phenylethynyl)tin were processed as in Example 31 to provide the designated compound.
MS (DCI/NH₃) m/e 221 (M+NH₄)⁺.

### Example 32B

### 6-[4-(phenylethynyl)phenyl]-1,3,5-triazine-2,4-diamine

4-(Phenylethynyl)benzonitrile was processed as in Example 1 to provide the title compound.
mp 248-249 °C;
MS (DCI/NH₃) m/e 289 (M+H)⁺:
¹H NMR (300 MHz, DMSO-d₆) δ 8.30 (d, 2H), 7.67 (d, 2H), 7.61-7.58 (m, 2H), 7.5-7.43 (m, 3H), 6.82 (br s, 4H);
Anal. calcd for C₁₇H₁₃N₅: C, 71.06; H, 4.56; N, 24.37. Found: C, 70.79; H, 4.73; N, 24.08.

### Example 33

### N,N'-(6-[1,1'-biphenyl]-4-yl-1,3,5-triazin-2,4-diyl)bis[acetamide]

### Example 33A

4-Phenylbenzonitrile was processed as in Example 1 to provide the designated compound.
MS (DCI/NH₃) m/e 264 (M+H)⁺.

### Example 33B

### N,N'-(6-[1,1'-biphenyl]-4-yl-1,3,5-triazin-2,4-diyl)bis[acetamide]

A solution of Example 33A (0.26g, 0.99 mmol) in acetic anhydride (10 mL) was refluxed for 20 hours and cooled to room temperature. The precipitate was rinsed with saturated NaHCO₃, and dried under vacuum to provide the title compound.
mp >260 °C;
MS (DCI/NH₃) m/e 348 (M+H)⁺;
¹H NMR (300 MHz, DMSO-d₆) δ 10.79 (s, 2H), 8.43 (d, 2H), 7.91 (d, 2H), 7.79 (d, 2H), 7.52 (m, 2H), 7.41 (m, 1H), 2.41 (s, 6H);
Anal. calcd for C₁₉H₁₇N₅O₂: C, 65.70; H, 4.93; N, 20.16. Found: C, 65.63; H, 4.84; N, 20.18.

### Example 34

### N-(4-amino-6-[1,1'-biphenyl]-4-yl-1,3,5-triazin-2-yl)acetamide

A solution of Example 33A (0.38g. 1.4 mmol) in acetic anhydride (4 mL) was heated at 80 °C for 20 hours, treated with ethyl acetate and cooled to room temperature. The precipitate was collected by vacuum filtration, rinsed with aqueous sodium carbonate, and dried under vacuum to yield the title compound.
mp >260 °C;
MS (DCI/NH₃) m/e 306 (M+H)⁺;
¹H NMR (300 MHz, DMSO-d₆) δ 10.22 (s, 1H), 8.39 (d, 2H), 7.83 (d. 2H), 7.77 (d, 2H), 7.53 (m, 3H), 7.41 (m, 2H), 2.36 (s, 3H);
Anal. calcd for C₁₇H₁₅N₅O·0.2CH₃CO₂H: C, 65.86; H, 5.02; N, 22.07. Found: C, 65.82; H, 4.97; N, 22.37.

### Example 35

### N-[4-(4,6-diamino-1,3,5-triazin-2-yl)phenyl]-1-naphthalenesulfonamide

Example 10A was processed as in Example 23 but substituting 1-naphthalenesulfonyl chloride for benzenesulfonyl chloride to provide the title compound.
mp >250 °C;
MS (DCI/NH₃) m/e 393 (M+H)⁺;
¹H NMR (300 MHz, DMSO-d₆) δ 10.8 (s, 1H), 8.8 (d, 1H), 8.3 (d, 1H), 8.2 (d, 1H), 8.1 (d, 1H), 8.0 (s, 1H), 7.83-7.6 (m, 4H), 7.2 (t, 1H), 7.15-7.1 (m, 1H), 6.83-6.7 (m, 4H);
Anal. calcd for C₁₉H₁₆N₆O₂S·H₂O: C, 55.59; H, 4.42; N, 20.47. Found: C, 55.57; H, 4.42; N, 20.52.

### Example 36

### 6-(4'-azido[1,1'-biphenyl]-4-yl)-1,3,5-triazine-2,4-diamine

### Example 36A

A solution of 4'-amino[1,1'-biphenyl]-4-carbonitrile (0.490 g, 2.53 mmol) in trifluoroacetic acid (12.5 mL) was treated sequentially with sodium nitrite (0.338 g, 4.90 mmol) and sodium azide (0.33 g. 5.1 mmol), stirred at room temperature for 10 minutes, treated with water and extracted with ethyl acetate. The extract was dried (MgSO₄), concentrated to provide the designated compound.
MS (DCI/NH₃) m/e 238 (M+NH₄)⁺.

### Example 36B

### 6-(4'-azido-[1,1'-biphenyl]-4-yl)-1,3,5-triazine-2,4-diamine

Example 36A was processed as in Example 1 to provide the title compound.
mp 230 °C (decomposes);
MS (DCI/NH₃) m/e 305 (M+H)⁺;
¹H NMR (300 MHz, DMSO-d₆) δ 8.32 (d, 2H), 7.79 (m, 4H), 7.24 (d, 2H), 6.74 (bds, 4H);
Anal. calcd for C₁₅H₁₂N₈·0.33H₂O: C, 58.07; H, 4.11; N, 36.12. Found: C, 58.15; H, 3.84; N, 33.09.

### Example 37

### 6-[4-(4-morpholinylsulfonyl)phenyl]-1,3,5-triazine-2,4-diamine

### Example 37A

A solution of 4-cyanobenzenesulfonyl chloride (600 mg, 2.98 mmol), morpholine (300 mg, 3.44 mmol), and pyridine (350 µL, 342 mg, 4.33 mmol) in dichloromethane (10 ml) was stirred overnight at room temperature, treated with saturated NH₄Cl and extracted with ethyl acetate. The extract was washed with water and brine, dried (MgSO₄) and concentrated to provide the designated compound.
MS (DCI/NH₃) m/e 270 (M+NH₄)⁺.

### Example 37B

### 4-(2,4-diamino-1,3,5-triazin-2-yl)-N-(4-morpholinyl)benzenesulfonamide

Example 37A was processed as in Example 1 to provide the title compound.
mp >260 °C;
MS (DCI/NH₃) m/e 337 (M+H)⁺;
¹H NMR (300 MHz, DMSO-d₆) δ 8.46 (d, 2H), 7.83 (d, 2H), 6.91 (br s, 4H), 3.65-3.60 (m, 4H), 2.94-2.88 (m, 4H);
Anal. calcd for C₁₃H₁₆N₆O₃S: C, 46.42; H, 4.79; N, 24.98. Found: C, 46.21; H, 4.69; N, 25.24.

### Example 38

### 6-[4-(2-furanyl)phenyl]-1,3,5-triazine-2,4-diamine

6-(4-Bromophenyl)-1,3,5-triazine-2,4-diamine was processed as in Example 31 but substituting 2-tri-n-butyltinfuran for 2-tri-n-butyltinthiophene to provide the title compound.
MS (DCI/NH₃) m/e 254 (M+H)⁺;
¹H NMR (300 MHz, DMSO-d₆) δ 8.3 (d, 2H), 7.8 (d, 3H), 7.05 (d, 1H), 6.8-6.7 (br s, 4H), 6.65-6.6 (m, 1H);
Anal. calcd for C₁₃H₁₁N₅O: C, 60.30; H, 5.57; N, 24.30. Found: C, 59.83; H, 5.44; N, 24.86.

### Example 39

### N,N'-[6-(4-phenoxyphenyl)-1,3,5-triazine-2,4-diyl]bis[acetamide]

Example 9 was processed as in Example 33B to provide the title compound.
mp 243-245 °C;
MS (DCI/NH₃) m/e 364 (M+H)⁺;
¹H NMR (300 MHz, DMSO-d₆) δ 10.74 (s, 2H), 8.38 (d, 2H), 7.47 (t, 2H), 7.24 (t, 1H), 7.14 (dd, 4H), 2.38 (s, 6H);
Anal. calcd for C₁₉H₁₇N₅O₃: C, 62.80; H, 4.72; N, 19.27. Found: C, 62.56; H, 4.82; N, 19.40.

### Example 40

### N-[4-amino-6-(4-phenoxyphenyl)-1,3,5-triazin-2-yl]acetamide

Example 9 was processed as in Example 34 to provide the title compound.
mp >260 °C;
MS (DCI/NH₃) m/e 322 (M+H)⁺;
¹H NMR (300 MHz, DMSO-d₆) δ 10.21 (s, 1H), 8.32 (d, 2H), 7.46 (t, 2H), 7.37 (bds, 2H), 7.13 (d, 2H), 7.08 (d, 2H), 2.32 (s, 3H);
Anal. calcd for C₁₇H₁₅N₅O₂: C. 63.54; H, 4.71; N, 21.79, Found: C, 63.25; H, 4.79; N, 21.84.

### Example 41

### 6-(5-phenyl-2-furanyl)-1,3,5-triazine-2,4-diamine

### Example 41A

Methyl 5-bromo-2-furoate, phenylboronic acid, and tetrakis(triphenylphosphine) palladium were processed as in Example 21 to provide the designated compound.
MS (DCI/NH₃) m/e 203 (M+H)⁺.

### Example 41B

### 6-(5-phenyl-2-furanyl)-1,3,5-triazine-2,4-diamine

Examples 41A and 2B were processed as in Example 2C to provide the title compound.
mp >260 °C;
MS (DCI/NH₃) m/e 254 (M+H)⁺;
¹H NMR (300 MHz, DMSO-d₆) δ 7.80 (d, 2H), 7.52-7.44 (m, 2H), 7.41-7.37 (m, 1H), 7.23 (dd, 1H), 7.16 (dd, 1H), 6.78 (br s, 4H);
Anal. calcd for C₁₃H₁₁N₅O: C, 61.65; H, 4.37; N, 27.65. Found: C, 61.33; H, 4.37; N, 27.42.

### Example 42

### 6-(5-phenyl-2-thienyl)-1,3,5-triazine-2,4-diamine

Methyl 5-phenylthiophene-2-carboxylate was processed as in Examples 41A and 41B to provide the title compound.
mp >250 °C;
MS (DCI/NH₃) m/e 270 (M+H)⁺;
¹H NMR (300 MHz, DMSO-d₆) δ 7.80 (d, 1H), 7.71-7.76 (m, 2H), 7.56 (d, 1H), 7.31-7.49 (m, 3H), 6.78 (bds, 4H);
Anal. calcd for C₁₃H₁₁N₅S·0.5H₂O: C, 56.09; H, 4.34; N, 25.16. Found: C, 56.35; H, 4.01; N, 25.27.

### Example 43

### N,N'-[6-(4-phenylcyclohexyl)-1,3,5-triazin-2,4-diyl]bis[acetamide]

Example 3 was processed as in Example 33B to provide the title compound.
mp 235-236 °C;
MS (DCI/NH₃) m/e 354 (M+H)⁺;
¹H NMR (300 MHz. DMSO-d₆) δ 10.61 (s, 2H), 7.30 (m, 4H), 7.18 (m, 1H), 2.63 (m,
1H), 2.56 (m, 1H), 2.36 (s, 6H), 1.98 (m, 4H), 1.63 (m, 4H);
Anal. calcd for C₁₉H₂₃N₅O₂·0.25H₂O: C, 63.76; H, 6.62; N, 19.57. Found: C, 63.83; H, 6.52; N, 19.27.

### Example 44

### N-[4-amino-6-(4-phenylcyclohexyl)-1,3,5-triazin-2-yl]acetamide

Example 3 was processed as in Example 34 to provide the title compound.
mp > 260 °C;
MS (DCI/NH₃) m/e 312 (M+H)⁺;
¹H NMR (300 MHz, DMSO-d₆) δ 10.02 (s, 1H), 7.28 (m, 7H), 2.54 (m, 1H), 2.44 (m, 1H), 2.25 (s, 3H), 1.96 (m, 4H), 1.59 (m, 4H);
Anal. calcd for C₁₇H₂₁N₅O: C, 65.57; H, 6.80; N, 22.49. Found: C, 65.37; H, 6.85; N, 22.74.

### Example 45

### 6-(4-phenyl-1-naphthalenyl)-1,3,5-triazine-2,4-diamine

### Example 45A

A solution of 4-methoxy-1-naphthalenecarbonitrile (3.5 g, 19 mmol) in dichloromethane (15 mL) at -78 °C was treated with BBr₃ (5 g, 20 mmol) in dichloromethane (15 mL), stirred at room temperature for 18 hours, treated with AlCl₃ (5 g, 38 mmol), stirred at room temperature for 18 hours, treated with water and extracted with ethyl acetate. The extract was washed with water and brine, dried (MgSO₄), and concentrated. The residue was purified by flash chromatography on silica gel with 30% ethyl acetate/hexane to provide the designated compound.
MS (DCI/NH₃) m/e 187 (M+NH₄)⁺.

### Example 45B

A solution of Example 45A (1.0 g, 5.9 mmol), triethylamine (1 mL, 7.2 mmol) and N-phenyl-trifluoromethanesulfonamide (2.1 g, 5.9 mmol) in dichloromethane (15 mL) at 0 °C was stirred overnight at room temperature. The reaction was treated with ethyl acetate and washed sequentially with 10% HCl, 20% KOH, water, and brine, dried (MgSO₄), and concentrated to provide the designated compound.
MS (DCI/NH₃) m/e 319 (M+NH₄)⁺.

### Example 45C

Example 45B and phenylboronic acid were processed as in Example 21 to provide the designated compound.
MS (DCI/NH₃) m/e 247 (M+NH₄)⁺.

### Example 45D

### 6-(4-Phenyl-1-napthalenyl)-1,3,5-triazine-2,4-diamine

Example 45C was processed as in Example 1 to provide the title compound.
mp 239-240 °C;
MS (DCI/NH₃) m/e 314 (M+H)⁺;
¹H NMR (300 MHz, DMSO-d₆) δ 8.84-8.80 (m, 1H), 7.97 (d, 1H), 7.85-7.81 (m. 1H), 7.69-7.48 (m, 8H), 6.84 (bds, 4H);
Anal. calcd for C₁₉H₁₅N₅: C, 72.82; H, 4.82; N, 22.34. Found: C, 72.68; H, 4.77; N, 22.35.

### Example 46

### 6-[4-(phenylthio)phenyl]-1,3,5-triazine-2,4-diamine

### Example 46A

A solution of 4-bromobenzonitrile (1.0 g, 5.5 mmol), thiophenol (644 mg, 5.8 mmol), K2C03 (1.9 g, 13.7 mmol) and CuI (1.05 g, 5.5 mmol) in DMF (20 mL) was heated at retlux for 24 hours, treated with ethyl acetate and filtered through Celite®. The filtrate was washed with water and brine, dried (MgSO₄), and concentrated. The residue was purified by flash chromatography on silica gel with 5% ethyl acetate/hexane to provide the designated compound.
MS (DCI/NH₃) m/e 229 (M+NH₄)⁺.

### Example 46B

### 6-[4-(phenylthio)phenyl]-1,3,5-triazine-2,4-diamine

Example 46A was processed as in Example 1 to provide the title compound.
mp 213-215 °C;
MS (DCI/NH₃) m/e 296 (M+H)⁺;
¹H NMR (300 MHz, DMSO-d₆) δ 8.19 (d, 2H), 7.44-7.41 (m, 5H), 7.32 (d, 2H), 6.77 (bds, 4H);
Anal. calcd for C₁₅H₁₃N₅S: C, 60.99; H, 4.43; N, 23.71. Found: C, 60.70; H, 4.32; N, 23.55.

### Example 47

### 6-(2-quinolinyl)-1,3,5-triazine-2,4-diamine

2-Quinolinecarbonitrile was processed as in Example 1 to provide the title compound.
MS (DCI/NH₃) m/e 239 (M+H)⁺;
¹H NMR (300 MHz, DMSO-d₆) δ 8.5 (d, 1H), 8.35 (d, 1H),. 8.15-8.0 (m, 2H), 7.9-7.8 (m, 1H), 7.75-7.7 (m, H), 7.1-7.0 (br s, 2H), 7.0-6.9 (br s, 2H);
Anal. calcd for C₁₂H₁₀N₆: C, 60.49; H, 4.23; N, 35.27. Found: C, 60.24; H, 3.94; N, 35.12.

### Example 48

### 6-(3-quinolinyl)-1,3,5-triazine-2,4-diamine

3-Quinolinecarbonitrile was processed as in Example 1 to provide the title compound.
mp > 250 °C;
MS (DCI/NH₃) m/e 239 (M+H)⁺;
¹H NMR (300 MHz, DMSO-d6) δ 9.7 (d, 1H), 9.1 (d, 1H),. 8.2-8.1 (m, 2H), 6.9-6.85 (m, 1H), 6.8-6.7 (m, 1H), 7.05-6.9 (br s, 4H);
Anal. calcd for C₁₂H₁₀N₆: C, 60.49; H, 4.23; N. 35.27. Found: C, 60.32; H, 4.06; N, 35.54.

### Example 49

### 6-(benzo[b]thien-2-ylmethyl)-1,3,5-triazine-2,4-diamine

Benzo[b]thiophene-2-acetonitrile was processed as in Example 1 to provide the title compound.
mp 216-218 °C;
MS (DCI/NH₃) m/e 258 (M+H)⁺;
¹H NMR (300 MHz, DMSO-d₆) δ 7.97-7.92 (m, 1H), 7.87-7.80 (m, 1H), 7.48 (s, 1H), 7.4-7.32 (m, 2H), 6.65 (br s, 4H), 3.90 (s, 2H);
Anal. calcd for C₁₂H₁₁N₅S: C, 56.01; H, 4.30; N, 27.21. Found: C, 55.97; H, 4.19; N, 27.31.

### Example 50

### 6-(2,2-dimethyl-2H-1-benzopyran-6-yl)-1,3,5-triazine-2,4-diamine

2,2-Dimethyl-2H-1-benzopyran-6-carbonitrile was processed as in Example 1 to provide the title compound.
MS (DCI/NH₃) m/e 270 (M+H)⁺;
¹H NMR (300 MHz, DMSO-d₆) δ 8.05 (dd, 1H), 7.95 (d, 1H), 6.9 (d, 1H), 6.78-6.75 (br s, 4H), 6.70 (d, 1H), 5.80 (d, 1H), 1.20 (s, 6H);
Anal. calcd for C₁₄H₁₅N₅O: C, 62.44; H, 5.61; N, 26.00. Found: C, 62.19; H, 5.70; N, 25.54.

### Example 51

### 6-(2,3-dihydro-1,4-benzodioxin-2-yl)-1,3,5-triazine-2,4-diamine

2,3-Dihydro-1,4-benzodioxine-2-carbonitrile was processed as in Example 1 to provide the title compound.
MS (DCI/NH₃) m/e 246 (M+H)⁺;
¹H NMR (300 MHz, DMSO-d₆) δ 7.0-6.75 (m, 8H), 3.5 (t, 1H), 3.3 (d, 2H);
Anal. calcd for C₁₁H₁₁N₅O₂: C, 53.81; H, 4.52; N, 28.55. Found: C, 53.80; H, 4.36; N, 28.40.

### Example 52

### 6-(tricyclo[3.3.1.1^{3.7}]decan-1-yl)-1,3,5-triazine-2,4-diamine

Methyl tricyclo[3.3.1.1^{3.7})decane-1-carboxylate and Example 2B were processed as in Example 2C to provide the title compound.
mp 261-262 °C;
MS (DCI/NH₃) m/e 246 (M+H)⁺;
¹H NMR (300 MHz, DMSO-d₆) δ 6.47 (br s, 4H), 2.05-1.95 (m, 3H), 1.9-1.88 (m, 6H), 1.77-1.60 (m, 6H);
Anal. calcd for C₁₃H₁₉N₅: C, 63.64; H, 7.80; N, 28.54. Found: C, 63.48; H, 7.66; N, 28.34.

### Example 53

### 6-(1-isoquinolinyl)-1,3,5-triazine-2,4-diamine

1-Isoquinolinecarbonitrile was processed as in Example 1 to provide the title compound.
mp >250°C;
MS (DCI/NH₃) m/e 239 (M+H)⁺;
¹H NMR (300 MHz, DMSO-d₆) δ 8.7 (d, 1H), 8.2 (d, 1H), 8.0 (d, 1H), 7.9 (d, 1H), 7.8 (dt, 1H), 7.65 (dt, 1H), 6.9 (bs, 4H);
Anal. calcd for C₁₂H₁₀N₆·0.3H₂O : C, 60.49; H, 4.23; N, 35.27. Found: C, 59.55; H, 4.35; N, 34.03.

### Example 54

### (+/-)-4-(4,6-diamino-1,3,5-triazine-2-yl)-α-phenylbenzenemethanol

A mixture of Example 11 (150 mg, 0.515 mmol) and sodium borohydride (6 mg, 0.15 mmol) in ethanol (5 mL) was heated at reflux for 30 minutes then stirred overnight at room temperature. The precipitate was rinsed with water and dried under vacuum to provide the title compound.
mp 214-216 °C;
MS (DCI/NH₃) m/e 294 (M+H)⁺;
¹H NMR (300 MHz, DMSO-d₆) δ 8.2 (d, 2H), 7.5 (d, 2H), 7.4 (d, 2H), 7.3 (t, 2H), 7.2 (m, 1H), 6.7 (br s, 4H), 6.0 (d, 1H), 5.75 (d, 1H);
Anal. calcd for C₁₆H₁₅N₅O: C, 65.51; H, 5.15; N, 23.87. Found: C, 65.33; H, 4.91; N, 23.65.

### Example 55

### 6-(2,3-dihydro-1,4-benzodioxin-6-yl)-1,3,5-triazine-2,4-diamine

2,3-Dihydro-1,4-benzodioxine-6-carbonitrile was processed as in Example 1 to provide the title compound.
mp 241-244 °C;
MS (DCI/NH₃) m/e 246 (M+H)⁺;
¹H NMR (300 MHz, DMSO-d₆) δ 8.8-8.75 (m, 2H), 6.95-6.9 (m, 1H), 6.9-6.8 (br s, 4H), 4.25-4.33 (m, 4H);
Anal. calcd for C₁₁H₁₁N₅O₂: C, 53.87; H, 4.52: N, 28.56. Found: C, 53.93; H, 4.27; N, 28.41.

### Example 56

### 6-(1-azabicyclo[2.2.2]octan-4-yl)-1,3,5-triazine-2,4-diamine

1-Azabicyclo[2.2.2]octane-4-carbonitrile was processed as in Example 1 to provide the title compound.
mp >245 °C;
MS (DCI/NH₃) m/e 221 (M+H)⁺;
¹H NMR (300 MHz. DMSO-d₆) δ 6.6-6.5 (br s, 4H), 3.35 (s, 2H), 2.9 (t, 5H), 1.7 (t, 5H); Anal. calcd for C₁₀H₁₆N₆: C, 54.53; H, 7.32; N, 38.15. Found: C, 54.40; H, 7.38; N, 38.25.

### Example 57

### 6-[4-(phenylsulfinyl)phenyl]1,3,5-triazine-2,4-diamine

A mixture of Example 49 (102 mg, 0.34 mmol) and Oxone® (106 mg, 0.17 mmol) in acetic acid (2 mL) was stirred overnight at ambient temperature, treated with saturated NaHCO₃ and extracted with ethyl acetate. The extract was washed with water and brine, dried (MgSO₄), and concentrated. The residue was recrystallized from ethanol to provide the title compound.
mp 253-255 °C;
MS (DCI/NH₃) m/e 312 (M+H)⁺;
¹H NMR (300 MHz, DMSO-d₆) δ 8.36 (d, 2H), 7.81 (d, 2H), 7.75-7.72 (m, 2H), 7.60-7.52 (m, 3H), 6.82 (br s, 4H);
Anal. calcd for C₁₅H₁₃N₅OS·0.25H₂O: C, 57.03; H, 4.30; N, 22.17. Found: C, 57.47; H, 4.04; N, 21.81.

### Example 58

### 6-[4-(phenylsulfonyl)phenyl]-1,3,5-triazine-2,4-diamine

4-(Phenylsulfonyl)benzonitrile (*J. Org. Chem.* **1989,** 54, 4691) was processed as in Example 1, to provide the title compound.
mp >250 °C;
MS (DCI/NH₃) m/e 328 (M+H)⁺;
¹H NMR (300 MHz, DMSO-d₆) δ 8.41 (d, 2H), 8.08 (d, 2H), 7.96 (d, 2H), 7.71-7.60 (m, 3H), 6.92 (br s, 4H);
Anal. calcd for C₁₅H₁₃N₅O₂S·0.25H₂O: C. 54.28; H, 4.10; N, 21.10. Found: C, 54.28; H, 3.92; N, 20.82.

### Example 59

### E/Z-[4-(4,6-diamino-1,3,5-triazine-2-yl)phenyl]phenylmethanone, oxime

A mixture of Example 11 (300 mg, 1.03 mmol) and hydroxylamine hydrochloride (70 mg, 1.0 mmol) in 1:1 ethanol/pyridine (10 mL) was heated at reflux for 3 hours, stirred overnight at room temperature, treated with water, and filtered. The precipitate was rinsed with water and dried to provide the title compound.
mp 97-107 °C;
MS (DCI/NH₃) m/e 307 (M+H)⁺;
¹H NMR (300 MHz, DMSO-d₆) δ 11.42 (s, 0.5H), 11.41 (s, 0.5H), 8.35 (d, 1H), 8.2 (d, 1H), 7.3-7.5 (m, 7H), 6.8 (br s, 4H);
Anal. calcd for C₁₆H₁₄N₆O·CH₃CH₂OH: C, 61.35; H, 5.72; N, 23.84. Found: C, 61.67; H, 5.29; N, 23.37.

### Example 60

### 6-pyrazinyl-1,3,5-triazine-2,4-diamine

Pyrazinecarbonitrile was processed as in Example 1 to provide the title compound.
mp>250 °C;
MS (DCINH₃) m/e 190 (M+H)⁺;
¹H NMR (300 MHz, DMSO-d₆) δ 6.9 (br s, 2H), 7.1 (br s, 2H), 8.75-8.8 (m, 2H), 9.3 (s, 1H);
Anal. calcd for C₇H₇N₇: C, 44.44; H, 3.72; N, 51.82. Found: C, 44.40; H, 3.62; N, 51.79.

### Example 61

### 2,4-diamino-6-[(4-phenylethenyl)phenyl]-1,3,5-triazine

### Example 61A

A solution of benzyltriphenylphosphonium chloride (22.8 g, 58 mmol) in THF (100 mL) at room temperature was treated with lithium hexamethyldisilazide (1M in toluene, 53 mL, 53 mmol), heated to reflux for 15 minutes, cooled to room temperature, treated with 4-cyanobenzaldehyde (7 g, 53 mmol) in THF (40 mL), stirred overnight at room temperature, acidified with 10% HCl, and filtered. The filtrate was extracted with ethyl acetate, dried (MgSO₄), and concentrated. The residue was dissolved in hot ethyl acetate and filtered through a plug of silica gel to provide the designated compound.

### Example 61B

Example 61A was processed as in Example 1 to provide the title compound.
mp 216-217 °C;
MS (DCI/NH₃) m/e 290 (M+H)⁺;
¹H NMR (300 MHz, DMSO-d₆) δ 8.25 (d, 2H), 7.75 (d, 2H), 7.65 (d, 2H), 7.2-7.4 (m, 5H), 6.75 (br s, 4H);
Anal. calcd for C₁₇H₁₅N₅·0.5CH₃CO₂CH₂CH₃: C, 68.45; H, 5.74; N, 21.00. Found: C, 68.50; H, 5.49; N, 21.43.

### Example 62

### 2,4-diamino-6-[(4-(2-nitrophenyl)ethenyl)phenyl]-1,3,5-triazine

4-Nitrobenzyltriphenylphosphonium bromide was processed as in Examples 61A and 61B to provide the title compound.
mp >250 °C;
MS (DCI/NH₃) m/e 335 (M+H)⁺;
¹H NMR (300 MHz, DMSO-d₆) δ 8.25 (t, 4H), 7.9 (d, 2H), 7.8 (d, 2H), 7.6 (m, 2H), 6.8 (br s, 4H);
Anal. calcd for C₁₇H₁₄N₆O₂: C, 61.07; H, 4.22; N, 25.14. Found: C, 60.78; H, 4.12; N, 24.89.

### Example 63

### 6-[1,1'-biphenyl]-4-yl-N,N'-dimethyl-1,3,5-triazine-2,4-diamine

### Example 63A

### 2-[1,1'-biphenyl]-4-yl-4,6-dichloro-1,3,5-triazine

A mixture of 4-phenyl-phenyl magnesium bromide (prepared from 4-bromobiphenyl (7.75 g, 33 mmol) and magnesium turnings (0.83 g, 35 mmol) in 40 mL ether) and cyanuric chloride (4.00 g, 21.7 mmol) in benzene (90 mL) was stirred at 0 °C for 90 minutes. The reaction was evaporated to dryness, and the residue was flash chromatographed on silica gel with 50% hexanes/methylene chloride to provide the desired compound (2.80 g, 43%).
MS (DCI/NH₃) m/e 301 (M+H)⁺.

### Example 63B

### 6-[1,1'-biphenyl]-4-yl-N,N'-dimethyl-1,3,5-triazine-2,4-diamine

A mixture of Example 63A (0.52 g, 1.72 mmol) and *N*-methylamine (30 mmol) in tetrahydrofuran (25 mL) was stirred at ambient temperature for 72 hours. The reaction was reduced in volume and diluted with water. The precipitate was collected, rinsed with water and ether, and dried. Purification by reverse phase HPLC provided the desired compound.
mp 198-200 °C;
MS (DCl/NH₃) m/e 292 (M+H)⁺;
¹H NMR (300 MHz, DMSO-d₆) δ 8.37 (m, 2H), 7.74 (m, 4H), 7.51 (m, 2H), 7.39 (m, 1H), 7.22 (bdm, 2H), 2.82 (m, 6H);
Anal. calcd for C₁₇H₁₇N₅•0.25 H₂O: C, 69.01; H, 5.96; N, 23.67. Found: C, 69.37; H, 5.85; N, 23.63.

### Example 64

### 6-[1,1'-biphenyl]-4-yl-N-methyl-1,3,5-triazine-2,4-diamine

### Example 64A

### 4-[1,1'-biphenyl]-4-yl-6-chloro-1,3,5-triazin-2-amine

A mixture of 2-[1,1'-biphenyl]-4-yl-4,6-dichloro-1,3,5-triazine (Example 63A) (0.804 g, 2.67 mmol) in 40 mL ether and concentrated ammonium hydroxide (2 mL, 30 mmol) in tetrahydrofuran (30 mL) was stirred at 0 °C for 60 minutes and at ambient temperature for 20 minutes. The reaction was reduced in volume, diluted with water, and the precipitate was collected, rinsed with water and ether, and dried to provide the desired compound (0.090 g, 12%).
MS (DCI/NH₃) m/e 282 (M+H)⁺.

### Example 64B

### 6-[1,1'-biphenyl]-4-yl-N-methyl-1,3,5-triazine-2,4-diamine

Example 64A (0.090 g, 0.32 mmol) and *N*-methylamine (6 mmol) in tetrahydrofuran (9 mL) was stirred at ambient temperature for 24 hours. The reaction was reduced in volume and diluted with water. The precipitate was collected, rinsed with water and ether, and dried to provide the desired compound (0.062 g, 70%).
mp 237-238 °C;
MS (DCI/NH₃) m/e 278 (M+H)⁺;
¹H NMR (300 MHz, DMSO-d₆) δ 8.39 (d, 1H), 8.32 (d, 1H), 7.77 (m, 4H), 7.51 (t, 2H), 7.41 (m, 1H), 7.25 (q, 1H), 6.79 (bds, 2H), 2.79 (d, 3H);
Anal. calcd for C₁₆H₁₅N₅•0.5 C₄H₈O₂: C, 67.27; H, 5.96; N, 21.79. Found: C, 67.20; H, 5.71; N, 22.05.

### Example 65

### 6-(bicyclo[2.2.1]hept-2-yl)-1,3,5-triazine-2,4-diamine

### Example 65A

### 6-(bicyclo[2.2.1]hept-2-en-5-yl)-1,3,5-triazine-2,4-diamine

Bicyclo[2.2.1]hept-2-ene-5-carbonitrile was processed as in Example 1 to provide the desired compound.
MS (DCI/NH₃) m/e 204 (M+H)⁺

### Example 65B

### 6-(bicyclo[2.2.1]hept-2-yl)-1,3,5-triazine-2,4-diamine

A solution of Example 65A in methanol was reduced with hydrogen gas and palladium on charcoal, filtered, and evaporated to provide the desired compound.
mp 216-217 °C;
MS (DCI/NH₃) m/e 206 (M+H)⁺;
¹H NMR (300 MHz, DMSO-d₆) δ 6.52 (bds, 4H), 2.83 (m, 1H), 2.39 (m, 1H), 2.21 (m, 1H). 2.04 (m, 1H), 1.91 (m, 1H), 1.6-1.2 (m, 5H), 6.52 (m, 1H);
Anal. calcd for C₁₀H₁₅N₅: C; 58.52, H; 7.37, N; 34.12. Found: C; 58.59, H; 7.40, N; 34.00.

### Example 66

### 6-[1,1'-biphenyl]-4-yl-N,N'-diethyl-1,3,5-triazine-2,4-diamine

A mixture of 2,4-di-N-ethylamino-6-chloro-1,3,5-triazine (0.55 g, 2.7 mmol) and tetrakis(triphenylphosphine) palladium (0.19 g, 0.16 mmol) in dry, degassed dimethylacetamide (45 mL) was heated to 100 °C, treated sequentially with 4-(phenyl)phenyl boronic acid (Yabroff et al., *Journal of the American Chemical Society,* Volume 56, **1934**, pp. 1850-1856) (0.80 g, 4.0 mmol) in absolute ethanol (15 mL) and saturated aqueous sodium bicarbonate (30 mL), and the reaction mixture was maintained at 100 °C for 3 days. The reaction mixture was cooled to room temperature and diluted with ethyl acetate. The organic layer was washed with brine, dried (MgSO₄), concentrated, and vacuum dried. The residue was recrystallized from 2:1 dioxane/ethanol to provide 0.15 g (17%) of the desired compound as a white solid.
mp 183-184 °C;
MS (DCI/NH₃) m/e 320 (M+H)⁺;
¹H NMR (300 MHz, DMSO-d₆) δ 8.36 (m, 2H), 7.78 (d, 2H), 7.73 (d, 2H), 7.49 (m, 2H), 7.38 (m, 1H), 7.28 (m, 2H), 3.40 (m, 4H), 1.16 (m, 6H);
Anal. calcd for C₁₉H₂₁N₅•0.2 C₄H₈O₂: C, 70.91; H, 6.55; N, 8.28. Found: C, 71.21; H, 6.50; N, 21.13.

### Example 67

### 6-(2'-nitro[1,1'-biphenyl]-4-yl)-1,3,5-triazine-2,4-diamine

4-Cyano-2'-nitrobiphenyl was processed as in Example 1 to provide the desired compound.
mp >250 °C;
¹H NMR (300 MHz, DMSO-d₆) δ 8.3 (d, 2H, J=9 Hz), 8.05 (dd, 1H), 7.8 (m, 1H), 7.6-7.7 (m, 2H), 7.45 (d, 2H), 6.8 (br s. 4H);
MS (DCI/NH₃) m/e 309 (M+H)⁺;
Anal. calcd for C₁₅H₁₂N₆O₂: C, 58.44; H, 3.92; N, 27.26. Found: C, 58.46; H, 3.99; N, 27.15.

### Example 68

### 6-(6-methyl-3-pyridinyl)-1,3,5-triazine-2,4-diamine

6-Methylnicotinonitrile was processed as in Example 1 to provide the desired compound.
mp >260 °C;
MS (DCI/NH₃) m/e 203 (M+H)⁺;
¹H NMR (300 MHz, DMSO-d₆) δ 9.23 (d, 1H), 8.39 (dd, 1H, J=11), 7.38 (d, 1H), 6.81 (br s, 4H), 2.56 (s, 3H);
Anal. calcd for C₉H₁₀N₆: C, 53.45; H, 4.98; N, 41.55. Found: C, 53.46; H, 4.94; N, 41.84.

### Example 69

### 6-(6-chloro-3-pyridinyl)-1,3,5-triazine-2,4-diamine

Methyl 6-chloronicotinate and imidodicarbonimidic diamide (2B) was processed as in Example 2C to provide the desired compound.
mp >260°C;
MS (DCI/NH₃) m/e 223, 225 (M+H)⁺;
¹H NMR (300 MHz, DMSO-d₆) δ 9.17 (d, 1H), 8.46 (dd, 1H, J=11), 7.62 (d, 1H), 6.91 (br s, 4H);
Anal. calcd for C₈H₇ClN₆: C, 43.15; H, 3.16; N, 37.74. Found: C, 43.05; H, 3.08; N, 37.50.

### Example 70

### 6-(5-bromo-3-pyridinyl)-1,3,5-triazine-2,4-diamine

5-Bromonicotinonitrile was processed as in Example 1 to provide the desired compound.
mp >260 °C;
MS (DCI/NH₃) m/e 267 (M+H)⁺;
¹H NMR (300 MHz, DMSO-d₆) δ 9.3 (d, 1H), 8.82 (d, 1H, J=3 Hz), 8.62-8.64 (m, 1H), 6.8-7.1 (brs, 1H);
Anal. calcd for C₈H₇BrN₆: C, 35.98; H, 2.64; N, 31.47. Found: C, 35.89; H, 2.53; N, 31.22.

### Example 71

### 6-(2,3-dihydro-2,2,3,3-tetrafluoro-1,4-benzodioxin-6-yl)-1,3,5-triazine-2,4-diamine

6-Cyano-2,3-dihydro-2,2,3,3-tetrafluoro-1,4-benzodioxane was processed as in Example 1 to provide the desired compound.
mp 176-179 °C;
MS (DCI/NH₃) m/e 275 (M+H)⁺;
¹H NMR (300 MHz, DMSO-d₆) δ 7.98 (d, 1H), 7.71 (d, 1H), 7.17 (dd, 1H), 7.16 (br s, 2H), 6.95 (br s, 2H), 3.85 (s, 3H);
Anal. calcd for C₁₁H₇F₄N₅O₂: C; 41.65, H; 2.22, N; 22.08. Found: C; 41.55, H; 2.10, N; 22.09.

### Example 72

### 6-[4-[(4-chlorophenyl)methoxy]phenyl]-1,3,5-triazine-2,4-diamine

4-[(4-Chlorophenyl)methoxy]benzonitrile was processed as in Example 1 to provide the desired compound.
mp 246-248 °C;
MS (DCI/NH₃) m/e 342 (M+H)⁺;
¹H NMR (300 MHz, DMSO-d₆) δ 7.45 (s, 4H), 7.25 (d, 2H), 6.9 (d, 2H), 6.6 (br s, 4H), 5.05 (s, 2H), 3.55 (s, 2H);
Anal. calcd for C₁₇H₁₆ClN₅O: C, 59.74; H, 4.72; N, 20.49. Found: C, 59.64; H, 4.64; N, 20.49.

### Example 73

### 6-[4-(1-piperidinylsulfonyl)phenyl]-1,3,5-triazine-2,4-diamine

### Example 73A

### 1-[(4-cyanophenyl)sulfonyl]piperidine

A mixture of 4-cyanobenzenesulfonyl chloride (0.51 g, 2.5 mmol) and piperidine (0.60 mL, 517 mg, 6.04 mmol) in 10 mL methylene chloride was stirred overnight at ambient temperature. The organic layer was washed successively with water, 5% HCl and brine, dried (Na₂SO₄) and concentrated. The resulting white solid (0.61 g, 96%) was used with no further purification.

### Example 73B

### 6-[4-(1-piperidinylsulfonyl)phenyl]-1,3,5-triazine-2,4-diamine

The product of Example 73A was processed as in Example 1 to provide the desired compound.
m.p. >260 °C;
MS (DCI/NH₃) m/e 335 (M+H)⁺;
¹H NMR (300 MHz, DMSO-d₆) δ 8.43 (d, 2H), 7.84 (d, 2H), 6.90 (bds, 4H), 2.90-2.97 (m, 4H), 1.50-1.59 (m, 4H), 1.32-1.42 (m, 2H);
Anal. calcd for C₁₄H₁₈N₆O₂S: C, 50.28; H, 5.42; N, 25.13. Found: C, 50.43; H, 5.32; N, 25.12.

### Example 74

### 6-(1-benzoyl-4-piperidinyl)-1,3,5-triazine-2,4-diamine

### Example 74A

### 1-benzoyl-4-piperidinecarbonitrile

A mixture of 1-benzoyl-4-piperidone (2.0 g, 9.8 mmol), tosylmethyl isocyanide (2.5 g, 12.8 mmol) and ethanol (1.0 mL, 17.1 mmol) in 30 mL DME was cooled in an ethanol/ice bath, and potassium tert-butoxide was added at such a rate to maintain the reaction temperature at <10 °C. The cold bath was removed, and the reaction was allowed to stir overnight at room temperature. The solids were removed by filtration, rinsed with DME, and the filtrate was evaporated. The residue was dissolved in EtOAc, washed with water and brine, dried (MgSO₄), filtered through silica gel, and concentrated to give 2.14 g (66%) of a slightly yellow oil.

### Example 74B

### 6-(1-benzoyl-4-piperidinyl)-1,3,5-triazine-2,4-diamine

The product of Example 74A was processed as in Example 1 to provide the desired compound.
m.p. 246-248 °C;
MS (DCI/NH₃) m/e 299 (M+H)⁺;
¹H NMR (300 MHz, DMSO-d₆) δ 7.43-7.49 (m, 3H), 7.33-7.39 (m, 2H), 6.58 (bds, 4H), 4.44-4.52 (bm, 1H), 3.55-3.67 (bm, 1H), 2.79-3.27 (bm, 2H), 1.53-1.94 (bm, 5H);
Anal. calcd for C₁₅H₁₈N₆O: C, 60.38; H, 6.08; N, 28.16. Found: C, 60.09; H, 6.02; N, 28.29.

### Example 75

### 6-[1-(phenylmethyl)-4-piperidinyl]-1,3,5-triazine-2,4-diamine

*N*-Benzyl-4-piperidone was processed as in example 74A and 74B to provide the desired compound.
m.p. >260 °C;
MS (DCI/NH₃) m/e 285 (M+H)⁺;
¹H NMR (300 MHz, DMSO-d₆) d 7.19-7.32 (m, 5H), 6.50 (bds, 4H), 3.44 (s, 2H), 2.78-2.86 (m, 2H), 2.16-2.28 (m, 1H), 1.90-1.99 (m, 2H), 1.63-1.76 (m, 4H);
Anal, calcd for C₁₅H₂₀N₆•H₂O: C, 59.58; H, 7.33; N, 27.79. Found; C, 60.06; H, 7.19; N, 27.94.

### Example 76

### N,N'-diacetyl-6-[4-(phenylsulfonyl)phenyl]-1,3,5-triazine-2,4-diamine

6-[4-(phenylsulfonyl)phenyl]1,3,5-triazine-2,4-diamine (Example 58) was processed as in Example 33B to provide the desired compound.
mp > 260 °C;
MS (DCI/NH₃) m/e 412 (M+H)⁺;
¹H NMR (300 MHz, DMSO-d₆) d 8.52 (d, 2H, J=8 Hz), 8.18 (d, 2H, J=8 Hz), 8.01 (m, 2H), 7.73 (m, 1H), 7.68 (m, 2H), 2.37 (s, 6H);
Anal. calcd for C₁₉H₁₇N₅O₄: C, 55.47; H, 4.16; N, 17.02. Found: C, 55.47; H, 4.19; N, 17.11.

### Example 77

### N-acetyl-6-[4-(phenylsulfonyl)phenyl]-1,3,5-triazine-2,4-diamine

6-[4-(phenylsulfonyl)phenyl]1,3,5-triazine-2,4-diamine (Example 58) was processed as in Example 34 to provide the desired compound.
mp > 260 °C;
MS (DCI/NH₃) m/e 370 (M+H)⁺;
¹H NMR (300 MHz, CF₃CO₂D) δ 8.51 (d, 2H), 8.27 (d, 2H), 8.06 (d, 2H), 7.78 (t, 1H), 7.68 (t, 2H), 2.56 (s, 3H);
Anal. calcd for C₁₇H₁₅N₅O₃•0.5 H₂O: C, 53.96; H, 4.26; N, 18.51. Found: C, 53.75; H, 3.91; N, 18.83.

### Example 78

### 6-[2-(1-piperidinyl)phenyl)]-1,3,5-triazine-2,4-diamine

2-(1-piperidinyl)benzonitrile was processed as in Example 1 to provide the desired compound.
mp >250°C;
MS (DCI/NH₃) m/e 271 (M+H)⁺;
¹H NMR (300 MHz, DMSO-d₆) δ 7.27 (d, 2H), 6.93 (d, 2H), 6.89 (m, 1H), 6.63 (bds, 4H), 3.88 (m, 4H), 1.47 (bdm, 6H);
Anal. calcd for C₁₄H₁₈N₆: C; 62.20, H; 6.71, N; 31.09. Found: C; 61.88, H; 6.36, N; 31.37.

## Claims

1. The use of a compound of formula (I), 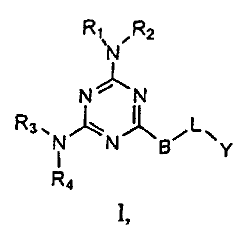 or a pharmaceutically acceptable salt thereof, for manufacturing a medicament for inhibiting angiogenesis in the treatment of cancer, diabetic retinopathy or macular degeneration in a mammal, wherein
R₁, R₂, R₃ and R₄ are independently selected from the group consisting of hydrogen, C₁-C₂₀-alkyl and C₁-C₂₀-alkanoyl;
B is selected from the group consisting of monocyclic aromatic carbocyclic ring, C₃-C₁₀-cycloalkyl, and heterocycle wherein heterocycle is selected from the group consisting of piperidinyl, thiazolyl, furanyl, and thienyl;
Y is selected from the group consisting of monocyclic aromatic carbocyclic ring, C₃-C₁₀-cycloalkyl, and heterocycle wherein heterocycle is selected from the group consisting of piperidinyl, morpholinyl, pyrrolyl, oxazolyl, thienyl, and pyridyl;
wherein the groups defining B and Y can be optionally substituted with 1-3 substituents independently selected from the group consisting of C₁-C₂₀-alkyl, C₁-C₂₀-alkoxy, amino, monocyclic aromatic carbocyclic ring, azido, cyano, halo, C₁-C₂₀-haloalkyl and nitro;
L is selected from the group consisting of a covalent bond, -C(=O)-, C₁-C₂₀-alkylene, C₂-C₂₀-alkenylene, C₂-C₂₀-alkynylene, NHC(O)NH-, -O-, and -S(O)ₜ-, wherein t is 0-2; or wherein said compound is 6-[1-(diphenylmethyl)-3-azetidinyl]-1,3,5-triazine-2,4-diamine.

2. The use of a compound according to Claim 1 in which R₁, R₂, R₃ and R₄ are hydrogen.

3. The use of a compound according to Claim 1 in which L is a covalent bond.

4. A compound of formula (I), 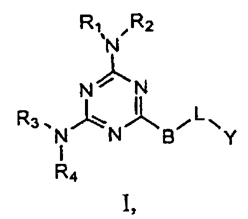 or a pharmaceutically acceptable salt thereof, wherein
R₁, R₂, R₃ and R₄ are independently selected from the group consisting of hydrogen, C₁-C₂₀-alkyl and C₁-C₂₀-alkanoyl;
B is selected from the group consisting of monocyclic aromatic carbocyclic ring, C₃-C₁₀-cycloalkyl, and heterocycle wherein heterocycle is selected from the group consisting of piperidinyl, thiazolyl, furanyl, and thienyl;
Y is selected from the group consisting of monocyclic aromatic carbocyclic ring, C₃-C₁₀-cycloalkyl, and heterocycle wherein heterocycle is selected from the group consisting of piperidinyl, morpholinyl, pyrrolyl, oxazolyl, thienyl, and pyridyl;
wherein the groups defining B and Y can be optionally substituted with 1-3 substituents independently selected from the group consisting of C₁-C₂₀-alkyl, C₁-C₂₀-alkoxy, amino, monocyclic aromatic carbocyclic ring, azido, cyano, halo, C₁-C₂₀-haloalkyl and nitro;
L is selected from the group consisting of a covalent bond, -C(=O)-, C₁-C₂₀-alkylene, C₂-C₂₀-alkenylene, C₂-C₂₀-alkynylene, NHC(O)NH-, -O-, and -S(O)ₜ-, wherein t is 0-2; or wherein said compound is 6-[1-(diphenylmethyl)-3-azetidinyl]-1,3,5-triazine-2,4-diamine; provided that 2,4-diamino-6-(5-chloro-2-cyclohexyloxy)phenyl)-1,3,5-triazine, 6-(1,1'-biphenyl-3-yl)-1,3,5-triazine-2,4-diamine and 6-(1,1'-biphenyl-4-yl)-1,3,5-triazine-2,4-diamine are excluded.

5. A compound of formula (I) 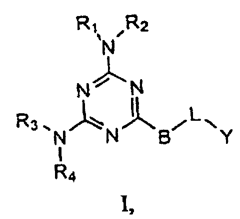 or a pharmaceutically acceptable salt thereof, for use as a therapeutic agent, wherein
R₁, R₂, R₃ and R₄ are independently selected from the group consisting of hydrogen, C₁-C₂₀-alkyl and C₁-C₂₀-alkanoyl;
B is selected from the group consisting of monocyclic aromatic carbocyclic ring, C₃-C₁₀-cycloalkyl, and heterocycle wherein heterocycle is selected from the group consisting of piperidinyl; thiazolyl, furanyl, and thienyl;
Y is selected from the group consisting of monocyclic aromatic carbocyclic ring, C₃-C₁₀-cycloalkyl, and heterocycle wherein heterocycle is selected from the group consisting of piperidinyl, morpholinyl, pyrrolyl, oxazolyl, thienyl, and pyridyl;
wherein the groups defining B and Y can be optionally substituted with 1-3 substituents independently selected from the group consisting of C₁-C₂₀-alkyl, C₁-C₂₀-alkoxy, amino, monocyclic aromatic carbocyclic ring, azido, cyano, halo, C₁-C₂₀-haloalkyl and nitro;
L is selected from the group consisting of a covalent bond, -C(=O)-, C₁-C₂₀-alkylene, C₂-C₂₀-alkenylene, C₂-C₂₀-alkynylene, NHC(O)NH-, -O-, and -S(O)ₜ-, wherein t is 0-2; or wherein said compound is 6-[1-(diphenylmethyl )-3-azetidinyl]-1,3,5-triazine-2,4-diamine; provided that 2,4-diamino-6-(5-chloro-2-cyclohexyloxy)phenyl)-1,3,5-triazine is excluded.

6. The compound of Claim 4 or 5 in which R₁, R₂, R₃ and R₄ are hydrogen.

7. The compound of Claim 4 or 5 in which L is a covalent bond.

8. A compound of Claim 4 selected from the group consisting of
6-[1-(diphenylmethyl)-3-azetidinyl]-1,3,5-triazine-2,4-diamine,
6-(1-phenyl-4-piperidinyl)-1,3,5-triazine-2,4-diamine,
trans-6-(4-phenylcyclohexyl)-1,3,5-triazine-2,4-diamine,
6-[3-(1H-pyrrol-1-yl)phenyl]-1,3,5-triazine-2,4-diamine,
6-[1,1'-biphenyl]-2-yl-1,3,5-triazine-2,4-diamine,
6-(4'-nitro[1,1'-biphenyl]-4-yl)-1,3,5-triazine-2,4-diamine,
6-[4-(4-pentylcyclohexyl)phenyl]-1,3,5-triazine-2,4-diamine,
6-(4-phenoxyphenyl)-1,3,5-triazine-2,4-diamine,
*N*-cyclohexyl-N'-[4-(4,6-diamino-1,3,5-triazin-2-yl)phenyl]urea,
*N*-[4-(4,6-diamino-1,3,5-triazin-2-yl)phenyl]-*N*'-phenyl urea,
6-(4'-pentyl[1,1'-biphenyl]-4-yl)-1,3,5-triazine-2,4-diamine,
6-[4'-(pentyloxy)[1,1'-biphenyl]-4-yl]-1,3,5-triazine-2,4-diamine,
6-(4'-amino[1,1'-biphenyl]-4-yl)-1,3,5-triazine-2,4-diamine,
6-[4-(5-oxazolyl)phenyl]-1,3,5-triazine-2,4-diamine,
6-[4-[[5-(trifluoromethyl)-2-pyridinyl]oxy]phenyl]-1,3,5-triazine-2,4-diamine,
4'-(4,6-diamino-1,3,5-triazine-2-yl)[1,1'-biphenyl]-4-carbonitrile,
6-(4'-methoxy[1,1'-biphenyl]-4-yl)-1,3,5-triazine-2,4-diamine,
6-(4'-fluoro[1,1'-biphenyl]-4-yl)-1,3,5-triazine-2,4-diamine
6-[1-([1,1'-biphenyl]-4-yl)-4-piperidinyl]-1,3,5-triazine-2,4-diamine,
6-(1-phenylcyclohexyl)-1,3,5-triazine-2,4-diamine,
6-[1-(4-methoxyphenyl)-4-piperidinyl]-1,3,5-triazine-2,4-diamine,
6-[2-[4-(trifluoromethyl)phenyl]-4-thiazolyl]-1,3,5-triazine-2,4-diamine,
6-[1-(4-methoxyphenyl)cyclohexyl]-1,3,5-triazine-2,4-diamine,
6-[4-(2-thienyl)phenyl]-1,3,5-triazine-2,4-diamine,
6-[4-(phenylethynyl)phenyl]-1,3,5-triazine-2,4-diamine,
*N,N*'-(6-[1,1'-biphenyl]-4-yl-1,3,5-triazin-2,4-diyl)bis[acetamide],
*N*-(4-amino-6-[1,1'-biphenyl]-4-yl-1,3,5-triazin-2-yl)acetamide,
6-(4'-azido[1,1'-biphenyl]-4-yl)-1,3,5-triazine-2,4-diamine,
6-[4-(4-morpholinylsulfonyl)phenyl]-1,3,5-triazine-2,4-diamine,
*N,N*'-[6-(4-phenoxyphenyl)-1,3,5-triazine-2,4-diyl]bis[acetamide],
*N*-[4-amino-6-(4-phenoxyphenyl)-1,3,5-triazin-2-yl]acetamide,
6-(5-phenyl-2-furanyl)-1,3,5-triazine-2,4-diamine,
6-(5-phenyl-2-thienyl)-1,3,5-triazine-2,4-diamine.
*N,N*'-[6-(4-phenylcyclohexyl)-1,3,5-triazin-2,4-diyl]bis[acetamide],
*N*-[4-amino-6-(4-phenylcyclohexyl)-1,3,5-triazin-2-yl]acetamide,
6-[4-(phenylthio)phenyl]-1,3,5-triazine-2,4-diamine,
6-[4-(phenylsulfinyl)phenyl]1,3,5-triazine-2,4-diamine,
6-[4-(phenylsulfonyl)phenyl]-1,3,5-triazine-2,4-diamine,
2,4-diamino-6-[(4-phenylethenyl)phenyl]-1,3,5-triazine,
2,4-diamino-6-[(4-(2-nitrophenyl)ethenyl)phenyl]-1,3,5-triazine,
6-[1,1'-biphenyl]-4-yl-*N*,*N*'-dimethyl-1,3,5-triazine-2,4-diamine,
6-[1,1'-biphenyl]-4-yl-*N*-methyl-1,3,5-triazine-2,4-diamine,
6-[1,1'-biphenyl]-4-yl-*N*,*N*'-diethyl-1,3,5-triazine-2,4-diamine,
6-(2'-nitro[1,1'-biphenyl]-4-yl)-1,3,5-triazine-2,4-diamine,
6-[4-(1-piperidinylsulfonyl)phenyl]-1,3,5-triazine-2,4-diamine,
6-(1-benzoyl-4-piperidinyl)-1,3,5-triazine-2,4-diamine,
6-[1-(phenylmethyl)-4-piperidinyl]-1,3,5-triazine-2,4-diamine,
*N*,*N*'-diacetyl-6-[4-(phenylsulfonyl)phenyl]-1,3,5-triazine-2,4-diamine,
*N*-acetyl-6-[4-(phenylsulfonyl)phenyl]-1,3,5-triazine-2,4-diamine, and
6-(2-piperidin-1-ylphenyl)-1,3,5-triazine-2,4-diamine;
or a pharmaceutically acceptable salt thereof.

9. A compound which is *trans*-6-(4-phenylcyclohexyl)-1,3,5-triazine-2,4-diamine, or a pharmaceutically acceptable salt thereof.

10. A pharmaceutical composition comprising a therapeutically effective amount of a compound of any one of Claims 5-9 and a pharmaceutically acceptable carrier.

11. The use of a compound of any one of Claims 5-7 for manufacturing a medicament for inhibiting angiogenesis in the treatment of diabetic retinopathy or macular degeneration in a mammal.

12. The use of a compound of Claim 8 or 9 for manufacturing a medicament for inhibiting angiogenesis in the treatment of diabetic retinopathy or macular degeneration in a mammal.

13. The use of a compound of any one of Claims 5-7 for manufacturing a medicament for inhibiting angiogenesis in the treatment of cancer in a mammal.

14. The use of a compound of Claim 8 or 9 for manufacturing a medicament for inhibiting angiogenesis in the treatment of cancer in a mammal.

## Patentansprüche

1. Die Verwendung einer Verbindung der Formel (I), 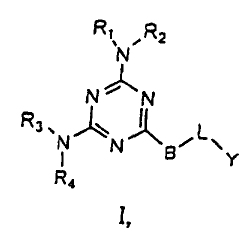 oder eines pharmazeutisch verträglichen Salzes davon, zur Herstellung eines Medikamentes zur Hemmung der Gefäßbildung bei der Behandlung von Krebs, diabetischer Retinopathie oder Makuladegeneration bei einem Säuger, worin R₁, R₂, R₃ und R₄ unabhängig gewählt sind aus der Gruppe bestehend aus Wasserstoff, C₁-C₂₀-Alkyl und C₁-C₂₀-Alkanoyl; B ist gewählt aus der Gruppe bestehend aus monocyclischem aromatischen carbocyclischen Ring, C₃-C₁₀-Cycloalkyl und Heterocyclus, wobei Heterocyclus gewählt ist aus der Gruppe bestehend aus Piperidinyl, Thiazolyl, Furanyl und Thienyl;
Y ist gewählt aus der Gruppe bestehend aus monocyclischem aromatischen carbocyclischen Ring, C₃-C₁₀-Cycloalkyl und Heterocyclus, wobei Heterocyclus gewählt ist aus der Gruppe bestehend aus Piperidinyl, Morpholinyl, Pyrrolyl, Oxazolyl, Thienyl und Pyridyl;
worin die Gruppen, die B und Y definieren, wahlweise substituiert sein können mit 1-3 Substituenten, die unabhängig gewählt sind aus der Gruppe bestehend aus C₁-C₂₀-Alkyl, C₁-C₂₀-Alkoxy, Amino, monocyclischem aromatischen carbocyclischen Ring, Azido, Cyano, Halo, C₁-C₂₀-Haloalkyl und Nitro;
L ist gewählt aus der Gruppe bestehend aus einer kovalenten Bindung, -C(=O)-, C₁-C₂₀-Alkylen, C₂-C₂₀-Alkenylen, C₂-C₂₀-Alkinylen, NHC(O)NH-, -O-, und -S(O)ₜ-, worin t 0-2 ist; oder worin die Verbindung 6-[1-(Diphenylmethyl)-3-azetidinyl]-1,3,5-triazin-2,4-diamin ist.

2. Die Verwendung einer Verbindung gemäß Anspruch 1, worin R₁, R₂, R₃ und R₄ Wasserstoff sind.

3. Die Verwendung einer Verbindung gemäß Anspruch 1, worin L eine kovalente Bindung ist.

4. Eine Verbindung der Formel (I), 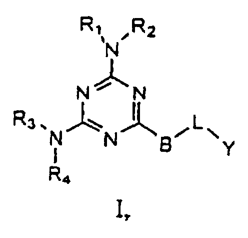 oder ein pharmazeutisch verträgliches Salz davon, worin R₁, R₂, R₃ und R₄ unabhängig gewählt sind aus der Gruppe bestehend aus Wasserstoff, C₁-C₂₀-Alkyl und C₁-C₂₀-Alkanoyl; B ist gewählt aus der Gruppe bestehend aus monocyclischem aromatischen carbocyclischen Ring, C₃-C₁₀-Cycloalkyl und Heterocyclus, wobei Heterocyclus gewählt ist aus der Gruppe bestehend aus Piperidinyl, Thiazolyl, Furanyl und Thienyl;
Y ist gewählt aus der Gruppe bestehend aus monocyclischem aromatischen carbocyclischen Ring, C₃-C₁₀-Cycloalkyl und Heterocyclus, wobei Heterocyclus gewählt ist aus der Gruppe bestehend aus Piperidinyl, Morpholinyl, Pyrrolyl, Oxazolyl, Thienyl und Pyridyl;
worin die Gruppen, die B und Y definieren, wahlweise substituiert sein können mit 1-3 Substituenten, die unabhängig gewählt sind aus der Gruppe bestehend aus C₁-C₂₀-Alkyl, C₁-C₂₀-Alkoxy, Amino, monocyclischem aromatischen carbocyclischen Ring, Azido, Cyano, Halo, C₁-C₂₀-Haloalkyl und Nitro;
L ist gewählt aus der Gruppe bestehend aus einer kovalenten Bindung , -C(=O)-, C₁-C₂₀-Alkylen, C₂-C₂₀-Alkenylen, C₂-C₂₀-Alkinylen, NHC(O)NH-, -O-, und -S(O)ₜ-, worin t 0-2 ist; oder worin die Verbindung 6-[1-(Diphenylmethyl)-3-azetidinyl]-1,3,5-triazin-2,4-diamin ist; vorausgesetzt dass 2,4-Diamino-6-(5-chlor-2-cyclohexyloxy)phenyl)-1,3,5-triazin, 6-(1,1'-Biphenyl-3-yl)-1,3,5-triazin-2,4-diamin und 6-(1,1'-Biphenyl-4-yl)-1,3,5-triazin-2,4-diamin ausgeschlossen sind.

5. Eine Verbindung der Formel (I), 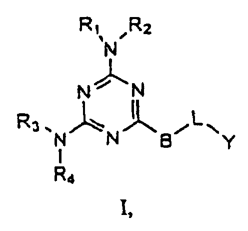 oder ein pharmazeutisch verträgliches Salz davon, zur Verwendung als ein therapeutisches Mittel, worin R₁, R₂, R₃ und R₄ unabhängig gewählt sind aus der Gruppe bestehend aus Wasserstoff, C₁-C₂₀-Alkyl und C₁-C₂₀-Alkanoyl; B ist gewählt aus der Gruppe bestehend aus monocyclischem aromatischen carbocyclischen Ring, C₃-C₁₀-Cycloalkyl und Heterocyclus, wobei Heterocyclus gewählt ist aus der Gruppe bestehend aus Piperidinyl, Thiazolyl, Furanyl und Thienyl;
Y ist gewählt aus der Gruppe bestehend aus monocyclischem aromatischen carbocyclischen Ring, C₃-C₁₀-Cycloalkyl und Heterocyclus, worin Heterocyclus gewählt ist aus der Gruppe bestehend aus Piperidinyl, Morpholinyl, Pyrrolyl, Oxazolyl, Thienyl und Pyridyl;
worin die Gruppen, die B und Y definieren, wahlweise substituiert sein können mit 1-3 Substituenten, die unabhängig gewählt sind aus der Gruppe bestehend aus C₁-C₂₀-Alkyl, C₁-C₂₀-Alkoxy, Amino, monocyclischem aromatischen carbocyclischen Ring, Azido, Cyano, Halo, C₁-C₂₀-Haloalkyl und Nitro;
L ist gewählt aus der Gruppe bestehend aus einer kovalenten Bindung, -C(=O)-, C₁-C₂₀-Alkylen, C₂-C₂₀-Alkenylen, C₂-C₂₀-Alkinylen, NHC(O)NH-, -O-, und -S(O)ₜ-, worin t 0-2 ist; oder worin die Verbindung 6-[1-(Diphenylmethyl)-3-azetidinyl]-1,3,5-triazin-2,4-diamin ist; vorausgesetzt dass 2,4-Diamino-6-(5-chlor-2-cyclohexyloxy)phenyl)-1,3,5-triazin ausgeschlossen ist.

6. Die Verbindung von Anspruch 4 oder 5, worin R₁, R₂, R₃ und R₄ Wasserstoff sind.

7. Die Verbindung von Anspruch 4 oder 5, worin L eine kovalente Bindung ist.

8. Eine Verbindung von Anspruch 4, gewählt aus der Gruppe bestehend aus
6-[1-(Diphenylmethyl)-3-azetidinyl]-1,3,5-triazin-2,4-diamin,
6-(1-Phenyl-4-piperidinyl)-1,3,5-triazin-2,4-diamin,
trans-6-(4-PhenylcycIohexyl)-1,3,5-triazin-2,4-diamin,
6-[3-(1H-Pyrrol-1-yl)phenyl]-1,3,5-triazin-2,4-diamin,
6-[1,1'-Biphenyl]-2-yl-1,3,5-triazin-2,4-diamin,
6-(4'-Nitro[1,1'-biphenyl]-4-yl)-1,3,5-triazin-2,4-diamin,
6-[4-(4-Pentylcyclohexyl)phenyl]-1,3,5-triazin-2,4-diamin,
6-(4-Phenoxyphenyl)-1,3,5-triazin-2,4-diamin,
*N*-Cyclohexyl-*N*'-[4-(4,6-diamino-1,3,5-iriazin-2-yl)phenyl]harnstoff,
*N*-[4-(4,6-Diamino-1,3,5-triazin-2-yl)phenyl]-*N*'-phenylharnstoff,
6-(4'-Pentyl[1,1'-biphenyl]-4-yl)-1,3,5-triazin-2,4-diamin,
6-[4'-(Pentyloxy)[1,1'-biphenyl]-4-yl]-1,3,5-triazin-2,4-diamin,
6-(4'-Amino[1,1'-biphenyl]-4-yl)-1,3,5-triazin-2,4-diamin,
6-[4-(5-OxazoIyl)phenyl]-1,3,5-triazin-2,4-diamin,
6-[4-[[5-(Trifluormethyl)-2-pyridinyl]oxy]phenyl]-1,3,5-triazin-2,4-diamin,
4'-(4,6-Diamino-1,3,5-triazin-2-yl)[1,1'-biphenyl]-4-carbonitril,
6-(4'-Methoxy[1,1'-biphenyl]-4-yl)-1,3,5-triazin-2,4-diamin,
6-(4'-Fluor[1,1'-biphenyl]-4-yl)-1,3,5-triazin-2,4-diamin,
6-[1-([1,1'-BiphenyI]-4-yl)-4-piperidinyl]-1,3,5-triazin-2,4-diamin,
6-(1-Phenylcyclohexyl)-1,3,5-triazin-2,4-diamin,
6-[1-(4-Methoxyphenyl)-4-piperidinyl]-1,3,5-triazin-2,4-diamin,
6-[2-[4-(Trifluormethyl)phenyl]-4-thiazolyl]-1,3,5-triazin-2,4-diamin,
6-[1-(4-Methoxyphenyl)cyclohexyl]-1,3,5-triazin-2,4-diamin,
6-[4-(2-Thienyl)phenyl]-1,3,5-triazin-2,4-diamin,
6-[4-(Phenylethinyl)phenyl]-1,3,5-triazin-2,4-diamin,
*N*,*N*'-(6-[1,1'-biphenyl]-4-yI-1,3,5-triazin-2,4-diyl)bis[acetamid],
*N*-(4-Amino-6-[1,1'-biphenyl]-4-yl-1,3,5-triazin-2-yl)acetamid,
6-(4'-Azido[1,1'-biphenyl]-4-yl)-1,3,5-triazin-2,4-diamin,
6-[4-(4-MorphoIinylsulfonyl)phenyl]-1,3,5-triazin-2,4-diamin,
*N,N*'-(6-(4-Phenoxyphenyl)-1,3,5-triazin-2,4-diyI]bis[acetamid],
*N*-[4-Amino-6-(4-phenoxyphenyl)-1,3,5-triazin-2-yl]acetamid,
6-(5-Phenyl-2-furanyl)-1,3,5-triazin-2,4-diamin,
6-(5-PhenyI-2-thienyl)-1,3,5-triazin-2,4-diamin,
*N,N*'-[6-(4-Phenylcyclohexyl)-1,3,5-triazin-2,4-diyl]bis[acetamid],
*N*-[4-Amino-6-(4-phenylcyclohexyl)-1,3,5-triazin-2-yl]acetamid,
6-[4-(Phenylthio)phenyl]-1,3,5-triazin-2,4-diamin,
6-[4-(Phenylsulfinyl)phenyl]-1,3,5-triazin-2,4-diamin,
6-[4-(Phenylsulfonyl)phenyl]-1,3,5-triazin-2,4-diamin,
2,4-Diamino-6-[(4-phenylethenyl)phenyl]-1,3,5-triazin,
2,4-Diamino-6-[(4-(2-nitrophenyl)ethenyl)phenyl]-1,3,5-triazin,
6-[1,1'-Biphenyl]-4-yl*-N,N'*-dimethyl-1,3,5-triazin-2,4-diamin,
6-[1,1'-Biphenyl]-4-yl-*N*-methyl-1,3,5-triazin-2,4-diamin,
6-[1,1'-Biphenyl]-4-yl-*N*,*N*'-diethyl-1,3,5-triazin-2,4-diamin,
6-(2'-Nitro[1,1'-biphenyl]-4-yl)-1,3,5-triazin-2,4-diamin,
6-[4-(1-Piperidinylsulfonyl)phenyl]-1,3,5-triazin-2,4-diamin,
6-(1-BenzoyI-4-piperidinyl)-1,3,5-triazin-2,4-diamin,
6-[1-(Phenylmethy])-4-piperidinyl]-1,3,5-triazin-2,4-diamin,
*N,N'*-Diacetyl-6-[4-(phenylsulfonyl)phenyl]-1,3,5-triazin-2,4-diamin,
*N*-Acetyl-6-[4-(phenylsulfonyl)phenyl]-1,3,5-triazin-2,4-diamin, und
6-(2-Piperidin-1-ylphenyl)-1,3,5-triazin-2,4-diamin;
oder ein pharmazeutisch verträgliches Salz davon.

9. Eine Verbindung, welche *trans*-6-(4-Phenylcyclohexyl)-1,3,5-triazin-2,4-diamin ist, oder ein pharmazeutisch verträgliches Salz davon.

10. Eine pharmazeutische Zusammensetzung, die eine therapeutisch wirksame Menge einer Verbindung von irgendeinem der Ansprüche 5-9 und einen pharmazeutisch verträglichen Träger umfasst.

11. Die Verwendung einer Verbindung von irgendeinem der Ansprüche 5-7 zur Herstellung eines Medikamentes zur Hemmung der Gefäßbildung bei der Behandlung von diabetischer Retinopathie oder Makuladegeneration bei einem Säuger.

12. Die Verwendung einer Verbindung von Anspruch 8 oder 9 zur Herstellung eines Medikamentes zur Hemmung der Gefäßbildung bei der Behandlung von diabetischer Retinopathie oder Makuladegeneration bei einem Säuger.

13. Die Verwendung einer Verbindung von irgendeinem der Ansprüche 5-7 zur Herstellung eines Medikamentes zur Hemmung der Gefäßbildung bei der Behandlung von Krebs bei einem Säuger.

14. Die Verwendung einer Verbindung von Anspruch 8 oder 9 zur Herstellung eines Medikamentes zur Hemmung der Gefäßbildung bei der Behandlung von Krebs bei einem Säuger.

## Revendications

1. Utilisation d'un composé de formule (I), 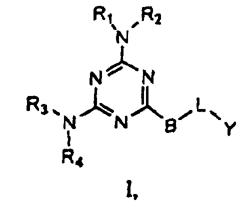 ou d'un sel pharmaceutiquement acceptable de celui-ci, pour la fabrication d'un médicament pour l'inhibition de l'angiogénèse dans le traitement du cancer, de la rétinopathie diabétique ou de la dégénérescence maculaire chez un mammifère, où
R₁, R₂, R₃ et R₄ sont, de façon indépendante, choisis dans le groupe constitué par l'hydrogène, un alkyle en C₁-C₂₀ et un alcanoyle en C₁-C₂₀ ;
B est choisi dans le groupe constitué par un cycle carbocyclique aromatique monocyclique, un cycloalkyle en C₃-C₁₀ et un hétérocycle, l'hétérocycle étant choisi dans le groupe constitué par le pipéridinyle, le thiazolyle, le furanyle et le thiényle ;
Y est choisi dans le groupe constitué par un cycle carbocyclique aromatique monocyclique, un cycloalkyle en C₃-C₁₀ et un hétérocycle, l'hétérocycle étant choisi dans le groupe constitué par le pipéridinyle, le morpholinyle, le pyrrolyle, l'oxazolyle, le thiényle et le pyridyle ;
les groupes définissant B et Y pouvant être, de façon optionnelle, substitués par 1 à 3 substituants choisis, de façon indépendante, dans le groupe constitué par un alkyle en C₁-C₂₀, un alcoxy en C₁-C₂₀, amino, un cycle carbocyclique aromatique monocyclique, azido, cyano, halogéno, un halogénoalkyle en C₁-C₂₀ et nitro ;
L est choisi dans le groupe constitué par une liaison covalente, -C(=O)-, un alkylène en C₁-C₂₀, un alcénylène en C₂-C₂₀, un alcynylène en C₂-C₂₀, NHC(O)NH-, -O-, et -S(O)ₜ-, où t vaut 0 à 2 ;
ou bien ledit composé est la 6-[1-(diphénylméthyl)-3-azétidinyl]-1,3,5-triazine-2,4-diamine.

2. Utilisation d'un composé selon la revendication 1, dans lequel R₁, R₂, R₃ et R₄ sont des atomes d'hydrogène.

3. Utilisation d'un composé selon la revendication 1, dans lequel L est une liaison covalente.

4. Composé de formule (I), 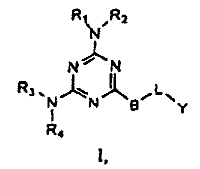 ou un sel pharmaceutiquement acceptable de celui-ci, où
R₁, R₂, R₃ et R₄ sont, de façon indépendante, choisis dans le groupe constitué par l'hydrogène, un alkyle en C₁-C₂₀ et un alcanoyle en C₁-C₂₀ ;
B est choisi dans le groupe constitué par un cycle carbocyclique aromatique monocyclique, un cycloalkyle en C₃-C₁₀ et un hétérocycle, l'hétérocycle étant choisi dans le groupe constitué par le pipéridinyle, le thiazolyle, le furanyle et le thiényle ;
Y est choisi dans le groupe constitué par un cycle carbocyclique aromatique monocyclique, un cycloalkyle en C₃-C₁₀ et un hétérocycle, l'hétérocycle étant choisi dans le groupe constitué par le pipéridinyle, le morpholinyle, le pyrrolyle, l'oxazolyle, le thiényle et le pyridyle ;
les groupes définissant B et Y pouvant être, de façon optionnelle, substitués par 1 à 3 substituants choisis, de façon indépendante, dans le groupe constitué par un alkyle en C₁-C₂₀, un alcoxy en C₁-C₂₀, amino, un cycle carbocyclique aromatique monocyclique, azido, cyano, halogéno, un halogénoalkyle en C₁-C₂₀ et nitro ;
L est choisi dans le groupe constitué par une liaison covalente, -C(=O)-, un alkylène en C₁-C₂₀, un alcénylène en C₂-C₂₀, un alcynylène en C₂-C₂₀, NHC(O)NH-, -O-, et -S(O)ₜ-, où t vaut 0 à 2 ;
ou bien ledit composé est la 6-[1-(diphénylméthyl)-3-azétidinyl]-1,3,5-triazine-2,4-diamine, sous réserve que la 2,4-diamino-6-(5-chloro-2-cyclohexyloxy)phényl)-1,3,5-triazine, la 6-(1,1'-biphényl-3-yl)-1,3,5-triazine-2,4-diamine et la 6-(1,1'-biphényl-4-yl)-1,3,5-triazine-2,4-diamine soient exclues.

5. Composé de formule (I), 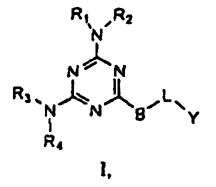 ou un sel pharmaceutiquement acceptable de celui-ci, pour son utilisation en tant qu'agent thérapeutique,
où
R₁, R₂, R₃ et R₄ sont, de façon indépendante, choisis dans le groupe constitué par l'hydrogène, un alkyle en C₁-C₂₀ et un alcanoyle en C₁-C₂₀ ;
B est choisi dans le groupe constitué par un cycle carbocyclique aromatique monocyclique, un cycloalkyle en C₃-C₁₀ et un hétérocycle, l'hétérocycle étant choisi dans le groupe constitué par le pipéridinyle, le thiazolyle, le furanyle et le thiényle ;
Y est choisi dans le groupe constitué par un cycle carbocyclique aromatique monocyclique, un cycloalkyle en C₃-C₁₀ et un hétérocycle, l'hétérocycle étant choisi dans le groupe constitué par le pipéridinyle, le morpholinyle, le pyrrolyle, l'oxazolyle, le thiényle et le pyridyle ;
les groupes définissant B et Y pouvant être, de façon optionnelle, substitués par 1 à 3 substituants choisis, de façon indépendante, dans le groupe constitué par un alkyle en C₁-C₂₀, un alcoxy en C₁-C₂₀, amino, un cycle carbocyclique aromatique monocyclique, azido, cyano, halogéno, un halogénoalkyle en C₁-C₂₀ et nitro ;
L est choisi dans le groupe constitué par une liaison covalente, -C(=O)-, un alkylène en C₁-C₂₀, alcénylène en C₂-C₂₀, alcyriylène en C₂-C₂₀, NHC(O)NH-, -O-, et -S(O)ₜ-, où t vaut 0 à 2 ;
ou bien ledit composé est la 6-[1-(diphénylméthyl)-3-azétidinyl]-1,3,5-triazine-2,4-diamine, sous réserve que la 2,4-diamino-6-(5-chloro-2-cyclohexyloxy)phényl)-1,3,5-triazine soit exclue.

6. Composé selon la revendication 4 ou 5, dans lequel R₁, R₂, R₃ et R₄ sont des atomes d'hydrogène.

7. Composé selon la revendication 4 ou 5, dans lequel L est une liaison covalente.

8. Composé selon la revendication 4 choisi dans le groupe constitué par
la 6-[1-(diphénylméthyl)-3-azétidinyl]-1,3,5-triazine-2,4-diamine,
la 6-(1-phényl-4-pipéridinyl)-1,3,5-triazine-2,4-diamine,
la trans-6-(4-phénylcyclohexyl)-1,3,5-triazine-2,4-diamine,
la 6-[3(1H-pyrrol-1-yl)phényl]-1,3,5-triazine-2,4-diamine,
la 6-[1,1'-biphényl]-2-yl-1,3,5-triazine-2,4-diamine,
la 6-(4'-nitro[1,1'-biphényl]-4-yl)-1,3,5-triazine-2,4-diamine,
la 6-[4-(4-pentylcyclohexyl)phényl]-1,3,5-triazine-2,4-diamine,
la 6-(4-phénoxyphényl)-1,3,5-triazine-2,4-diamine,
la N-cyclohexyl-N'-[4-(4,6-diamino-1,3,5-triazin-2-yl)phényl]urée,
la N-[4-(4,6-diamino-1,3,5-triazin-2-yl)phényl]-N'-phénylurée,
la 6-(4'-pentyl[1,1'-biphényl]-4-yl)-1,3,5-triazine-2,4-diamine,
la 6-(4'-(pentyloxy)[1,1'-biphényl]-4-yl)-1,3,5-triazine-2,4-diamine,
la 6-(4'-amino[1,1'-biphényl]-4-yl)-1,3,5-triazine-2,4-diamine,
la 6-[4-(5-oxazolyl)phényl]-1,3,5-triazine-2,4-diamine,
la 6-[4-[[5-(trifluorométhyl)-2-pyridinyl]oxy]phényl]-1,3,5-triazine-2,4-diamine,
le 4'-(4,6-diamino-1,3,5-triazine-2-yl)[1,1'-biphényl]-4-carbonitrile,
la 6-(4'-méthoxy[1,1'-biphényl]-4-yl)-1,3,5-triazine-2,4-diamine,
la 6-(4'-fluoro[1,1'-biphényl]-4-yl)-1,3,5-triazine-2,4-diamine,
la 6-[1-([1,1'-biphényl]-4-yl)-4-pipéridinyl]-1,3,5-triazine-2,4-diamine,
la 6-(1-phénylcyclohexyl)-1,3,5-triazine-2,4-diamine,
la 6-[1-(4-méthoxyphényl)-4-pipéridinyl]-1,3,5-triazine-2,4-diamine,
la 6-[2-[4-(trifluorométhyl)phényl]-4-thiazoly1]-1,3,5-triazine-2,4-diamine,
la 6-[1-(4-méthoxyphényl)cyclohexyl]-1,3,5-triazine-2,4-diamine,
la 6-[4-(2-thiényl)phényl]-1,3,5-triazine-2,4-diamine,
la 6-[4-(phényléthynyl)phényl]-1,3,5-triazine-2,4-diamine,
le N,N'-(6-[1,1'-hiphényl]-4-yl-1,3,5-triazin-2,4-diyl)bis[acétamide],
le N-(4-amino-6-[1,1'-biphényl]-4-yl-1,3,5-triazin-2-yl)acétamide,
la 6-(4'-azido[1,1'-biphényl]-4-yl)-1,3,5-triazin-2,4-diamine,
la 6-[4-(4-morpholinylsulfonyl)phényl]-1,3,5-triazine-2,4-diamme,
le N,N'-(6-(4-phénoxyphényl)-1,3,5-triazin-2,4-diyl)bis[acétamide],
le N-(4-amino-6-(4-phénoxyphényl)-1,3,5-triazin-2-yl)acétamide,
la 6-(5-phényl-2-furanyl)-1,3,5-triazine-2,4-diamine,
la 6-(5-phényl-2-thiényl)-1,3,5-triazine-2,4-diamine,
le N,N'-(6-(4-phénylcyclohexyl)-1,3,5-triazin-2,4-diyl)bis[acétamide],
le N-(4-amino-6-(4-phénylcyclohexyl)-1,3,5-triazin-2-yl)acétamide,
la 6-[4-(phénylthio)phényl]-1,3,5-triazine-2,4-diamine,
la 6-[4-(phénylsulfinyl)phényl]-1,3,5-triazine-2,4-diamine,
la 6-[4-(phénylsulfonyl)phényl]-1,3,5-triazine-2,4-diamine,
la 2,4-diamino-6-[(4-phényléthényl)phényl]-1,3,5-triazine,
la 2,4-diamino-6-[(4-(2-nitrophényl)éthényl)phényl]-1,3,5-triazine,
la 6-[1,1'-biphényl]-4-yl-N,N'-diméthyl-1,3,5-triazine-2,4-diamine,
la 6-[1,1'-biphényl]-4-yl-N-méthyl-1,3,5-triazine-2,4-diamine,
la 6-[1,1'-biphényl]-4-yl-N,N'-diéthyl-1,3,5-triazine-2,4-diamine,
la 6-(2'-nitro[1,1'-biphényl]-4-yl)-1,3,5-triazine-2,4-diamine,
la 6-[4-(1-pipéridinylsulfonyl)phényl]-1,3,5-triazine-2,4-diamine,
la 6-(1-benzoyl-4-pipéridinyl)-1,3,5-triazine-2,4-diamine,
la 6-[1-(phénylméthyl)-4-pipéridinyl]-1,3,5-triazine-2,4-diamine,
la N,N'-diacétyl-6-[4-(phénylsulfonyl)phényl]-1,3,5-triazine-2,4-diamine,
la N-acétyl-6-[4-(phénylsulfonyl)phényl]-1,3,5-triazine-2,4-diamine et
la 6-(2-pipéridin-1-ylphényl]-1,3,5-triazine-2,4-diamine ;
ou un sel pharmaceutiquement acceptable de celui-ci.

9. Composé qui est la trans-6-(4-phénylcyclohexyl)-1,3,5-triazine-2,4-diamine ou un sel pharmaceutiquement acceptable de celle-ci.

10. Composition pharmaceutique comprenant une quantité thérapeutiquement efficace d'un composé selon une quelconque des revendications 5 à 9 et un support pharmaceutiquement acceptable.

11. Utilisation d'un composé selon l'une quelconque des revendications 5 à 7, pour la fabrication d'un médicament pour l'inhibition de l'angiogénèse dans le traitement de la rétinopathie diabétique ou de la dégénérescence maculaire chez un mammifère.

12. Utilisation d'un composé selon la revendication 8 ou 9, pour la fabrication d'un médicament pour l'inhibition de l'angiogénèse dans le traitement de la rétinopathie diabétique ou de la dégénérescence maculaire chez un mammifère.

13. Utilisation d'un composé selon l'une quelconque des revendications 5 à 7 pour la fabrication d'un médicament pour l'inhibition de l'angiogénèse dans le traitement du cancer chez un mammifère.

14. Utilisation d'un composé selon la revendication 8 ou 9, pour la fabrication d'un médicament pour l'inhibition de l'angiogénèse dans le traitement du cancer chez un mammifère.
